(19) Europäisches Patentamt
European Patent Office
Office européen des brevets

(11) **EP 2 618 882 B1**

(12) **EUROPEAN PATENT SPECIFICATION**

(45) Date of publication and mention of the grant of the patent:
**26.12.2018 Bulletin 2018/52**

(21) Application number: **11849707.2**

(22) Date of filing: **26.08.2011**

(51) Int Cl.:
*A61B 34/30* *(2016.01)*    *A61B 90/00* *(2016.01)*
*A61B 34/20* *(2016.01)*    *A61B 18/12* *(2006.01)*

(86) International application number:
**PCT/US2011/049346**

(87) International publication number:
**WO 2012/082193 (21.06.2012 Gazette 2012/25)**

(54) **PROXIMITY SENSOR INTERFACE IN A ROBOTIC CATHETER SYSTEM**

NÄHERUNGSSENSORSCHNITTSTELLE IN EINEM ROBOTISCHEN KATHETERSYSTEM

INTERFACE DE CAPTEUR DE PROXIMITÉ DANS UN SYSTÈME DE CATHÉTER ROBOTISÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2010 US 970500**

(43) Date of publication of application:
**31.07.2013 Bulletin 2013/31**

(73) Proprietor: **St. Jude Medical Atrial Fibrillation Division Inc.**
**St. Paul, Minnesota 55117-9913 (US)**

(72) Inventors:
 • **SANDHU, Kulbir S.**
 **Singapore 279332 (SG)**
 • **SHAQUER, Cem**
 **Los Gatos, California 95030 (US)**

(74) Representative: **Kramer Barske Schmidtchen Patentanwälte PartG mbB**
**European Patent Attorneys**
**Landsberger Strasse 300**
**80687 München (DE)**

(56) References cited:
**US-A1- 2007 156 123    US-A1- 2009 247 993**
**US-A1- 2010 069 921    US-A1- 2010 069 921**
**US-A1- 2010 125 284    US-A1- 2010 256 558**

**Description**

BACKGROUND OF THE INVENTION

a. Field of the Invention

The present disclosure relates generally to a robotic control and guidance system (RCGS) for a medical device, and more particularly to a proximity/contact sensor interface in an RCGS.

b. Background Art

Electrophysiology (EP) catheters are used in a variety of diagnostic and/or therapeutic medical procedures to correct conditions such as atrial arrhythmia, including for example, ectopic atrial tachycardia, atrial fibrillation, and atrial flutter. Arrhythmia can create a variety of dangerous conditions including irregular heart rates, loss of synchronous atrioventricular contractions and stasis of blood flow which can lead to a variety of ailments.
In a typical EP procedure, a physician manipulates a catheter through a patient's vasculature to, for example, a patient's heart. The catheter typically carries one or more electrodes that may be used for mapping, ablation, diagnosis, and the like. Once at the target tissue site, the physician commences diagnostic and/or therapeutic procedures, for example, ablative procedures such as radio frequency (RF), microwave, cryogenic, laser, chemical, acoustic/ultrasound or high-intensity focused ultrasound (HIFU) ablation, to name a few different sources of ablation energy. The resulting lesion, if properly located and sufficiently contiguous with other lesions, disrupts undesirable electrical pathways and thereby limits or prevents stray electrical signals that can lead to arrhythmias. Such procedures require precise control of the catheter during navigation to and delivery of therapy to the target tissue site, which can invariably be a function of a user's skill level.
Robotic catheter systems are known to facilitate such precise control. Robotic catheter systems generally carry out (as a mechanical surrogate) input commands of a clinician or other end-user to deploy, navigate and manipulate a catheter and/or an introducer or sheath for a catheter or other elongate medical instrument, for example, a robotic catheter system described, depicted, and/or claimed in U.S. Application No. 12/347,811 (published as US2009247993) entitled "ROBOTIC CATHETER SYSTEM," owned by the common assignee of the present disclosure. Such robotic catheter systems include a variety of actuation mechanisms, such as electric motors, for controlling translation and deflection of the catheter and associated sheath. Such systems typically employ control algorithms for controlling the motion of the catheter based at least in part upon end-user input(s). While the location of the catheter, vis-à-vis target tissue site(s), can be monitored by a physician with a manually guided catheter, introducing a mechanically guided catheter system places a premium on patient safety. Despite these advancements, conventional systems still rely on the physician to determine what actions, if any, to take when the catheter is too close to the heart tissue.
There is therefore a need for improved systems and methods that enhance clinician control while reducing potential risk(s) in performing robotically-driven cardiac catheter procedures and thereby minimizes or eliminates one or more problems related thereto.
US 2010/0256558 A1 relates to a robotic catheter system and method for automated control of a catheter and related components, wherein actuation signals for navigating a medical device are not generated so as to navigate the medical device with a reduced deflection determined in accordance with deflection step control parameter indicative of a decrease in a deflection angle of a distal end of the medical device.
US 2010/0069921 A1 relates to a system relevant for the present invention.

BRIEF SUMMARY OF THE INVENTION

One advantage of the apparatus described, depicted and claimed herein relates to the reliable detection of the proximity and active control of the medical device in relation to body tissue to avoid unintended device-to-tissue contact in a graduated manner.
The invention is defined by the appended claims. The disclosure is directed to an apparatus for use in a robotic control system of the type suitable for manipulating a medical device in a body of a patient. The apparatus includes an electronic control unit (ECU) and a memory coupled to the ECU. Control logic is stored in the memory and is configured to be executed by the ECU. The control logic is configured to produce an actuation control signal to control actuation of a manipulator assembly portion of the robotic control system. The actuation control signal is produced so as to result in the navigation of the medical device in, and with respect to, a plurality of proximity zones in the body of the patient. The control logic is further configured to generate the actuation control signal, and thus control the navigation of the medical device, based on a so-called proximity signal. The proximity signal is indicative of a relative, distance between the medical device and body tissue. The effect the proximity signal has on the navigation of the medical device depends on certain navigation-altering attributes associated with each pre-defined proximity zone, and whether the medical device is in a particular zone based on the proximity signal.

In an embodiment, the control logic is arranged to control the navigation (*i.e.,* via the robotic control system) of the medical device based on a pre-planned movement, which can have a pre-planned path and a pre-planned deployment or retraction speed or velocity (*e.g.,* an automated catheter motion). The control logic is configured to make modifications to the pre-planned movement based on the proximity signal. The modifications can include one of (i) a reduced speed relative to the pre-planned speed and (ii) a stoppage of the device before completion of the pre-planned path. For example, when the device unexpectedly approaches an anatomical structure, the proximity signal indicates that the device will soon be or is currently "too close" to the approaching structure (*i.e.,* a so-called RED proximity zone). Under this circumstance, the control logic terminates the operating power to the actuation units (*e.g.,* electric motors) in the robotic control system, thereby stopping movement of the device. This response action prevents unintended device-to-tissue contact, which can have undesirable consequences (*e.g.,* insult to cardiac wall tissue or a coronary vein or the like). As a further example, when the device unexpectedly approaches an anatomical structure, like in the first example, but is somewhat further away, the proximity signal will so indicate this relationship (*i.e.,* a so-called YELLOW proximity zone). The control logic will reduce the navigation speed relative to the pre-planned (*i.e.,* normal or default) speed. This reduction in speed is an appropriate response action to avoid contact. When the medical device is in a still further proximity zone, a so-called GREEN proximity zone, which is farther still from any body tissue than the YELLOW proximity zone, no modifications to the pre-planned movement of the medical device are warranted. The apparatus also provides a user interface to allow a user to specify many of the parameters that define the characteristics of each proximity zone as well as the resulting response action when a medical device enters such proximity zone.

The foregoing and other aspects, features, details, utilities, and advantages of the present disclosure will be apparent from reading the following description and claims, and from reviewing the accompanying drawings.

BRIEF DESCRIPTION OF THE DRAWINGS

Figure 1 is an isometric diagrammatic view of a robotic catheter system, illustrating an exemplary layout of various system components.

Figure 2 is a side view of a manipulator assembly shown in Figure 1, coupled to a robotic support structure, showing side views of catheter and sheath manipulation mechanisms.

Figures 3a-3b are isometric views of a manipulator assembly shown in Figure 2, showing the catheter and sheath manipulation mechanism in greater detail.

Figures 4a-4c are isometric views showing a sheath manipulation base of Figures 3a-3b in greater detail.

Figures 5a-5b are isometric views showing a sheath cartridge of Figures 3a-3b in greater detail.

Figure 6 is a diagrammatic view of the sheath manipulation mechanism of Figure 2.

Figure 7 is an exemplary system for determining a proximity/contact signal.

Figure 8 is a schematic diagram illustrating how complex impedance is determined, which in turn can be used to compute an electrical coupling index (ECI) as a proximity signal.

Figure 9 is an electrode-to-tissue distance versus ECI diagram.

Figure 10 is a block diagram of an apparatus for use in an RCGS for detecting proximity/contact and automatically taking predetermined appropriate action.

Figure 11 is a block diagram showing, in greater detail, user interface logic and control logic used in the apparatus of Figure 10.

Figure 12 is a diagrammatic view of the user interface of Figure 11 for obtaining proximity zone parameters.

Figure 13 is a diagrammatic view of plural proximity zones.

Figure 14 is a flowchart showing a method of monitoring for and detecting proximity zone violations.

DETAILED DESCRIPTION OF THE INVENTION

Before proceeding to a detailed description of the proximity/contact sensor interface for a robotic catheter system, a brief overview (for context) of an exemplary robotic control and guidance system (RCGS) for manipulating a medical device will first be described. The description of the RCGS will detail how several electric motors can be used to control the translation, distal bending and virtual rotation of a catheter and surrounding sheath. After the description of the RCGS, the present specification will then provide a brief description of proximity/contact sensing technology that can be used in certain embodiments. Then, the present specification will describe the proximity/contact sensor interface for use in an RCGS.

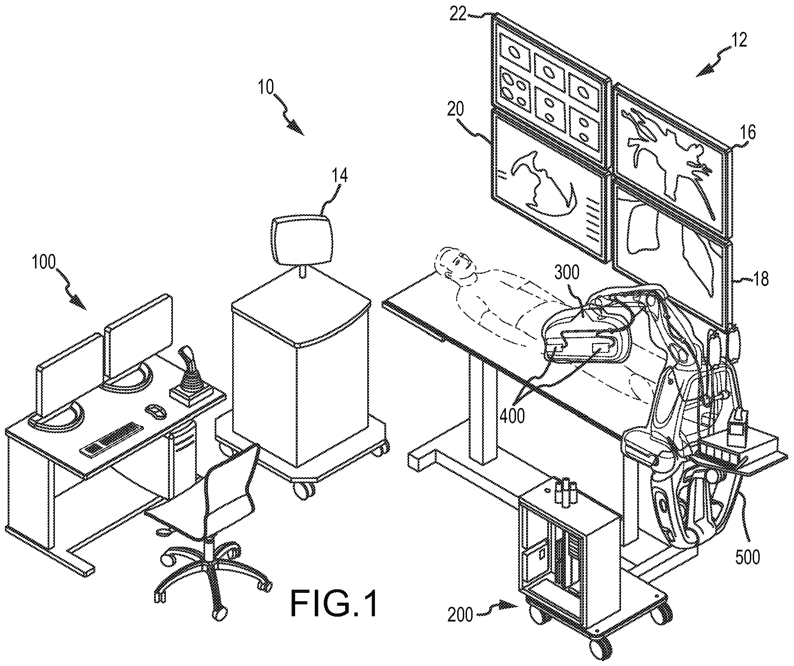

Now referring to the drawings wherein like reference numerals are used to identify identical components in the various views, Figure 1 is a diagrammatic view of an exemplary RCGS 10, in which several aspects of a system and method for automatic detection and prevention of motor runaway can be used.

*Exemplary RCGS System Description.* RCGS 10 can be likened to power steering for a catheter system. The RCGS 10 can be used, for example, to manipulate the location and orientation of catheters and sheaths in a heart chamber or in another body cavity or lumen. The RCGS 10 thus provides the user with a similar type of control provided by a conventional manually-operated system, but allows for repeatable, precise, and dynamic movements. For example, a user such as an electrophysiologist can identify locations (potentially forming a path) on a rendered computer model of the cardiac anatomy. The system can be configured to relate those digitally selected points to positions within a patient's actual/physical anatomy, and can thereafter command and control the movement of the catheter to the defined positions. Once at the specified target position, either the user or the system can perform the desired diagnostic or therapeutic function. The RCGS 10 enables full robotic navigation/guidance and control.

As shown in Figure 1, the RCGS 10 can generally include one or more monitors or displays 12, a visualization, mapping and navigation (including localization) system 14, a human input device and control system (referred to as "input control system") 100, an electronic control system 200, a manipulator assembly 300 for operating a device cartridge 400, and a manipulator support structure 500 for positioning the manipulator assembly 300 in proximity to a patient or a patient's bed.

Displays 12 are configured to visually present to a user information regarding patient anatomy, medical device location or the like, originating from a variety of different sources. Displays 12 can include (1) an ENSITE VELOCITY™ monitor 16 (coupled to system 14-described more fully below) for displaying cardiac chamber geometries or models, displaying activation timing and voltage data to identify arrhythmias, and for facilitating guidance of catheter movement; (2) a fluoroscopy monitor 18 for displaying a real-time x-ray image or for assisting a physician with catheter movement; (3) an intracardiac echo (ICE) display 20 to provide further imaging; and (4) an EP recording system display 22.

The system 14 is configured to provide many advanced features, such as visualization, mapping, navigation support and positioning (*i.e.*, determine a position and orientation (P&O) of a sensor-equipped medical device, for example, a P&O of a distal tip portion of a catheter). Such functionality can be provided as part of a larger visualization, mapping and navigation system, for example, an ENSITE VELOCITY system running a version of NavX™ software commercially available from St. Jude Medical, Inc., of St. Paul, Minnesota and as also seen generally by reference to U.S. Patent No. 7,263,397 entitled "METHOD AND APPARATUS FOR CATHETER NAVIGATION AND LOCATION AND MAPPING IN THE HEART" to Hauck et al., owned by the common assignee of the present disclosure,. System 14 can comprise conventional apparatus known generally in the art, for example, the ENSITE VELOCITY system described above or other known technologies for locating/navigating a catheter in space (and for visualization), including for example, the CARTO visualization and location system of Biosense Webster, Inc., (*e.g.,* as exemplified by U.S. Patent No. 6,690,963 entitled "System for Determining the Location and Orientation of an Invasive Medical Instrument", the AURORA® system of Northern Digital Inc., a magnetic field based localization system such as the gMPS system based on technology from MediGuide Ltd. of Haifa, Israel and now owned by St. Jude Medical, Inc. (*e.g.,* as exemplified by U.S. Patent Nos. 7,386,339, 7,197,354 and 6,233,476,) or a hybrid magnetic field-impedance based system, such as the CARTO 3 visualization and location system of Biosense Webster, Inc. (*e.g.,* as exemplified by U.S. Patent No. 7,536,218, and 7,848,789). Some of the localization, navigation and/or visualization systems can involve providing a sensor for producing signals indicative of catheter location and/or orientation information, and can include, for example one or more electrodes in the case of an impedance-based localization system such as the ENSITE VELOCITY system running NavX software, which electrodes can already exist in some instances, or alternatively, one or more coils (*i.e.,* wire windings) configured to detect one or more characteristics of a low-strength magnetic field, for example, in the case of a magnetic-field based localization system such as the gMPS system using technology from MediGuide Ltd. described above.

The input control system 100 is configured to allow a user, such as an electrophysiologist, to interact with the RCGS 10, in order to control the movement and advancement/withdrawal of both a catheter and sheath (*see, e.g.,*

commonly assigned U.S. Patent Application No. 12/751,843 filed 3/31/2010 entitled "ROBOTIC CATHETER SYSTEM" (docket no. 0G-043516US) and PCT/US2009/038597 entitled "ROBOTIC CATHETER SYSTEM WITH DYNAMIC RESPONSE" (docket no. 0G-043513WO), published as WO 2009/120982;). Generally, several types of input devices and related controls can be employed, including, without limitation, instrumented traditional catheter handle controls, oversized catheter models, instrumented user-wearable gloves, touch screen display monitors, 2-D input devices, 3-D input devices, spatially detected styluses, and traditional joysticks. For a further description of exemplary input apparatus and related controls, see, for example, commonly assigned U.S. Patent Application No. 12/933,063 entitled "ROBOTIC CATHETER SYSTEM INPUT DEVICE" (docket no. 0G-043527US) and U.S. Patent Application No. 12/347,442 (published as WO2009120948) entitled "MODEL CATHETER INPUT DEVICE" (docket no. 0G-043508US),. The input devices can be configured to directly control the movement of the catheter and sheath, or can be configured, for example, to manipulate a target or cursor on an associated display.

The electronic control system 200 is configured to translate (*i.e.,* interpret) inputs (*e.g.,* motions) of the user at an input device or from another source into a resulting movement of the catheter and/or surrounding sheath. In this regard, the system 200 includes a programmed electronic control unit (ECU) in communication with a memory or other computer readable media (memory) suitable for information storage. Relevant to the present disclosure, the electronic control system 200 is configured, among other things, to issue commands (*i.e.,* actuation control signals) to the manipulator assembly 300 (*i.e.,* to the actuation units-electric motors) to move or bend the catheter and/or sheath to prescribed positions and/or in prescribed ways, all in accordance with the received user input and a predetermined operating strategy programmed into the system 200. In addition to the instant description, further details of a programmed electronic control system can be found in commonly assigned U.S. Patent Application No. 12/751,843 filed 3/31/2010 entitled "ROBOTIC CATHETER SYSTEM" (docket no. 0G-043516US), described above. It should be understood that although the exemplary ENSITE VELOCITY System 14 and the electronic control system 200 are shown separately, integration of one or more computing functions can result in a system including an ECU on which can be run both (i) various control and diagnostic logic pertaining to the RCGS 10 and (ii) the visualization, mapping and navigation functionality of system 14.

The manipulator assembly 300, in response to such commands, is configured to maneuver the medical device (*e.g.,* translation movement, such as advancement and withdrawal of the catheter and/or sheath), as well as to effectuate distal end (tip) deflection and/or rotation or virtual rotation. In an embodiment, the manipulator assembly 300 can include actuation mechanisms/units (*e.g.,* a plurality of electric motor and lead screw combinations, or other electric motor configurations, as detailed below) for linearly actuating one or more control members (*e.g.,* steering wires) associated with the medical device for achieving the above-described translation, deflection and/or rotation (or virtual rotation).

A device cartridge 400 is provided for each medical device controlled by the RCGS 10. For this exemplary description of an RCGS, one cartridge is associated with a catheter and a second cartridge is associated with an outer sheath. The cartridge is then coupled, generally speaking, to the RCGS 10 for subsequent robotically-controlled movement.

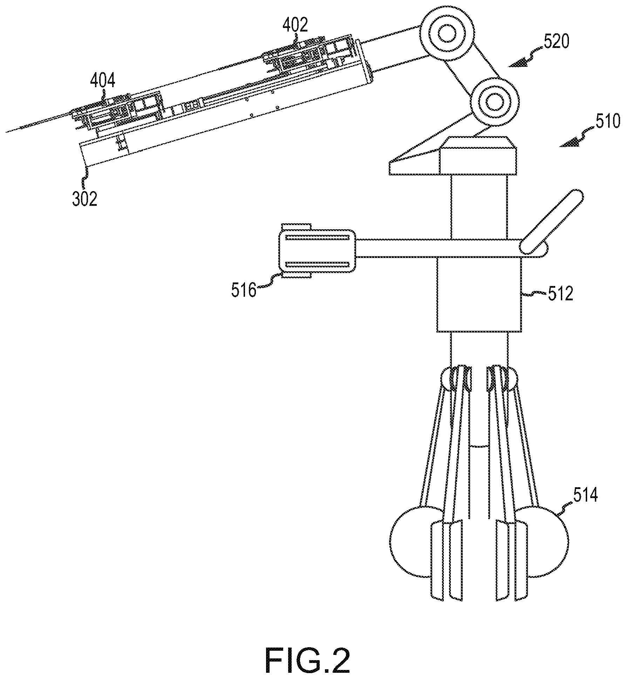

Figure 2 is a side view of an exemplary robotic catheter manipulator support structure, designated structure 510. The structure 510 can generally include a support frame 512 including retractable wheels 514 and attachment assembly 516 for attachment to an operating bed (not shown). A plurality of support linkages 520 can be provided for accurately positioning one or more manipulator assemblies, such as manipulator assembly 302. The assembly 302 is configured to serve as the interface for the mechanical control of the movements or actions of one or more device cartridges, such as catheter and sheath cartridges 402, 404 described below. Each device cartridge is configured to receive and retain a respective proximal end of an associated medical device (*e.g.,* catheter or sheath). The assembly 302 also includes a plurality of manipulation bases onto which the device cartridges are mounted. After mounting, the manipulator assembly 302, through the manipulation bases, is capable of manipulating the attached catheter and sheath.

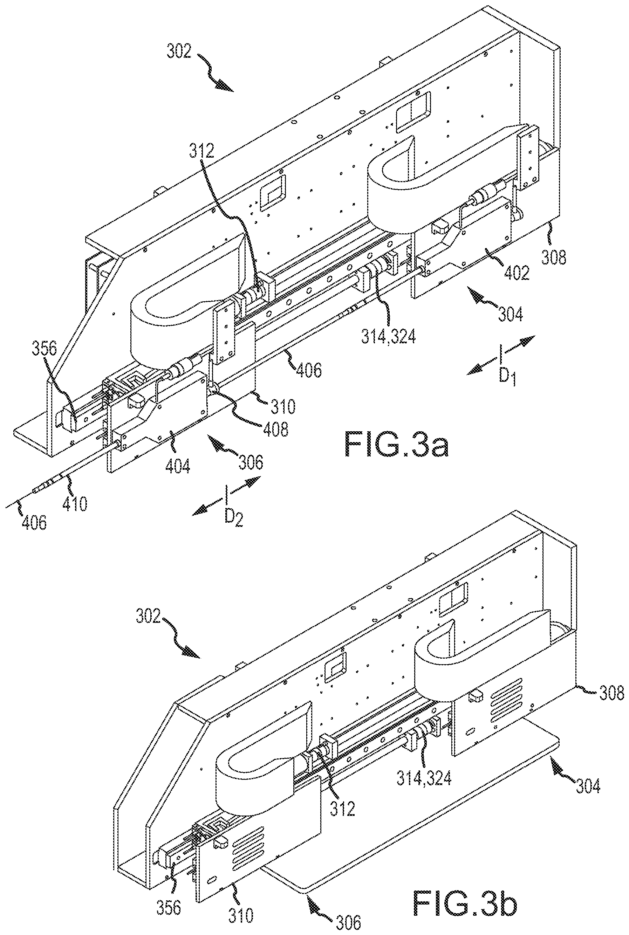

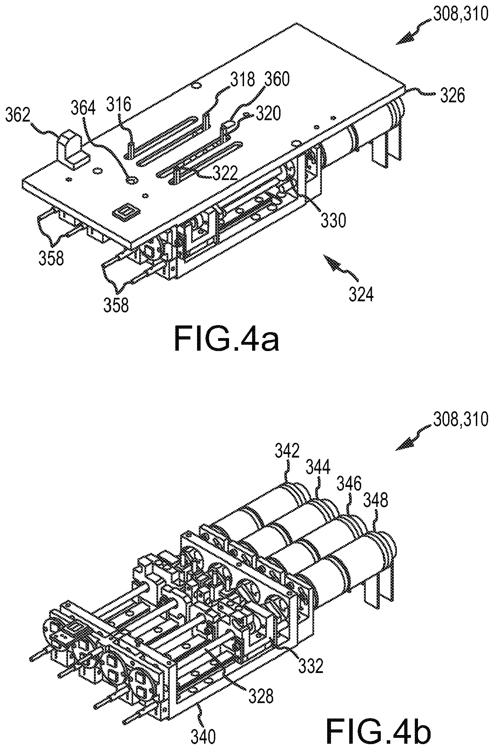

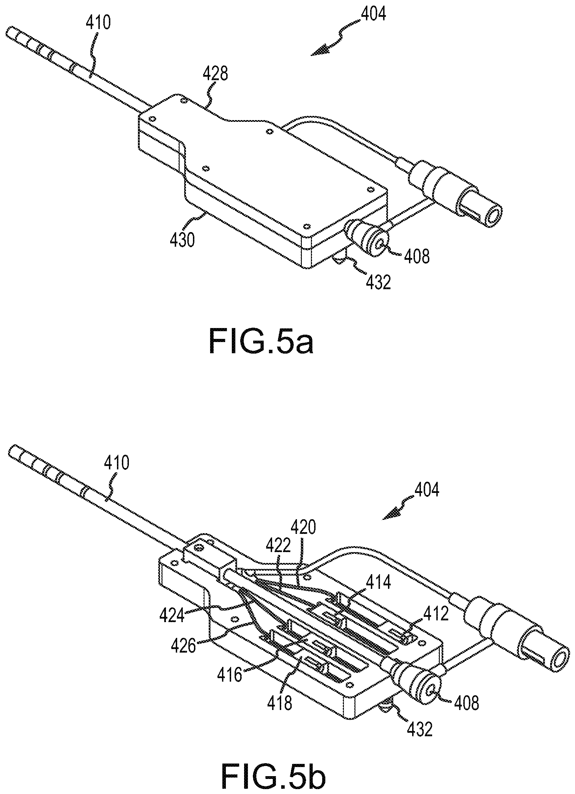

In the Figures to follow, Figures 3a-3b will show a manipulator assembly, Figures 4a-4c will show a manipulation base, and Figures 5a-5b will show a device cartridge.

Figure 3a is an isometric view, with portions omitted for clarity, of manipulator assembly 302. Assembly 302 includes a catheter manipulator mechanism 304, a sheath manipulator mechanism 306, a catheter manipulation base 308, a sheath manipulation base 310, a first (catheter) drive mechanism 312, a second (sheath) drive mechanism 314, and a track 356. As further shown, assembly 302 further includes a catheter cartridge 402 and a sheath cartridge 404, with a catheter 406 having a proximal end opening 408 coupled to the catheter cartridge 402 and a sheath 410 coupled to the sheath cartridge 404.

Catheter and sheath manipulator mechanisms 304, 306 are configured to manipulate the several different movements of the catheter 406 and the sheath 410. First, each mechanism 304, 306 is configured to impart translation movement to the catheter 406 and the sheath 410. Translation movement here refers to the independent advancement and retraction (withdrawal) as shown generally in the directions designated D1 and D2 in Fig. 3a. Second, each mechanism 304, 306 is also configured to effect deflection of the distal end of either or both of the catheter and sheath 406, 410. Third, each mechanism 304, 306 can be operative to effect a so-called virtual (omni-directional) rotation of the distal end portion of the catheter 406 and the sheath 410. Virtual rotation, for example, can be made through the use of independent four-wire steering control for each device (*e.g.,* eight total steering wires, comprising four sheath control wires and four catheter control wires). The distal end movement is referred to as "virtual" rotation because the outer surface of the sheath (or catheter) does not in fact rotate in the conventional sense (*i.e.,* about a longitudinal axis) but rather achieves the same movements as conventional uni-planar deflection coupled with axial rotation. In addition to the present description of virtual rotation, further details can be found in PCT/US2009/038597 entitled "ROBOTIC CATHETER SYSTEM WITH DYNAMIC RESPONSE" (docket no. 0G-043513WO), published as WO 2009/120982.

Each manipulator mechanism 304, 306 further includes a respective manipulation base 308, 310 onto which are received catheter and sheath cartridges 402, 404. Each interlocking base 308, 310 can be capable of travel in the longitudinal direction of the catheter/sheath (*i.e.,* D1, D2 respectively) along a track 356. In an embodiment, D1 and D2 can each represent a translation of approximately 8 linear inches (20.3 cm). Each interlocking base 308, 310 can be translated by a respective high precision drive mechanism 312, 314. Such drive mechanisms can include, for example and without limitation, an electric motor driven lead screw or ball screw.

The manipulator mechanisms 304, 306 are aligned with each other such that catheter 406 can pass through sheath 410 in a coaxial arrangement. Thus, sheath 410 can include a water-tight proximal sheath opening 408. Overall, the manipulator mechanisms 304, 306 are configured to allow not only coordinated movement but also relative movement between catheter and sheath cartridges 402, 404 (and thus relative movement between catheter and sheath).

Figure 3b is an isometric view of manipulator assembly 302, substantially the same as Figure 3a except that catheter and sheath cartridges 402, 404 are omitted (as well as catheter and sheath 406, 410) so as to reveal an exposed face of the manipulation bases 308, 310.

Figure 4a is an isometric, enlarged view showing manipulation base 308 (and base 310) in greater detail. Each cartridge 402, 404 has an associated manipulation base 308, 310. Each base 308, 310 can include a plurality of fingers 316, 318, 320 and 322 (*e.g.,* one per steering wire) that extend or protrude upwardly to contact and interact with steering wire slider blocks (*i.e.,* such as slider blocks 412, 414, 416, 418 are best shown in Fig. 5b) to independently tension select steering wires 420, 422, 424, 426 (also best shown in Fig. 5b). Each finger can be configured to be independently actuated (*i.e.,* moved back and forth within the oval slots depicted in Figure 4a) by a respective precision drive mechanism, such as a motor driven ball screw 324. A plate 326 provides a surface onto which one of the cartridges 402, 404 are seated.

Figure 4b is an isometric, enlarged view of base 308 (and base 310), substantially the same as Figure 4a except with plate 326 omitted. Each motor-driven ball screw 324 (best shown in Fig. 4a, *i.e.,* for both finger control and for cartridge translation control), can further include encoders to measure a relative and/or an absolute position of each element of the system. Moreover, each motor-driven ball screw 324 (*i.e.,* for both finger control and cartridge translation control) can be outfitted with steering wire force sensors to measure a corresponding steering wire tension. For example, a corresponding finger 316, 318, 320 or 322 can be mounted adjacent to a strain gauge for measuring the corresponding steering wire tension. Each motor-driven ball screw 324 can include a number of components, for example only, a rotary electric motor (*e.g.,* motors 342, 344, 346 and 348), a lead screw 328, a bearing 330 and a coupler 332 mounted relative to and engaging a frame 340. In the depicted embodiments linear actuation is primarily, if not exclusively, employed.

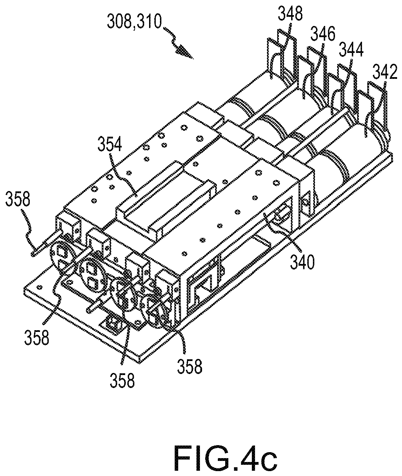

Figure 4c is an isometric, enlarged view of base 308 (and base 310) that is taken from an opposite side as compared to Figures 4a-4b. Bases 308, 310 can include components such as a plurality of electrically-operated motors 342, 344, 346 and 348, respectively coupled to fingers 316, 318, 320 and 322. A bearing 354 can be provided to facilitate the sliding of bases 308, 310 on and along track 356. A plurality of inductive sensors (*e.g.* home sensors) 358 can also be provided for guiding each manipulation base to a home position.

Figure 5a is an isometric, enlarged view showing, in greater detail, sheath cartridge 404. It should be understood that the description of sheath cartridge 404, except as otherwise stated, applies equally to catheter cartridge 402. Catheter 406 and sheath 410 can be substantially connected or affixed to respective cartridges 402, 404 (*e.g.,* in the neck portion). Thus, advancement of cartridge 404 correspondingly advances the sheath 410 and retraction of cartridge 404 retracts the sheath 410. Likewise, although not shown, advancement of cartridge 402 correspondingly advances catheter 406 while a retraction of cartridge 402 retracts catheter 406. As shown, sheath cartridge 404 includes upper and lower cartridge sections 428, 430.

Figure 5b is an isometric, enlarged view showing, in greater detail, sheath cartridge 404, with upper section 428 omitted to reveal interior components. Cartridge 404 can include slider blocks (*e.g.,* as shown for cartridge 404, slider blocks 412, 414, 416, 418), each rigidly and independently coupled to a respective one of a plurality of steering wires (*e.g.,* sheath steering wires 420, 422, 424, 426) in a manner that permits independent tensioning of each steering wire. Likewise, cartridge 402 for catheter 406 also includes slider blocks for coupling to a plurality (*i.e.,* four) steering wires. Device cartridges 402, 404 can be provided as a disposable item that is capable of being easily positioned (*e.g.,* snapped) into place (*i.e.,* onto a respective base 408, 410). Sheath cartridge 404 can be designed in a similar manner as the catheter cartridge 402, but will typically be configured to provide for the passage of catheter 406.

Referring to Figures 4a and 5a, catheter and sheath cartridges 402, 404 are configured to be secured or locked down onto respective manipulation bases 308, 310. To couple cartridge 402 (and 404) with base 308 (and 310), one or more locking pins (*e.g.,* 432 in Fig. 5a) on the cartridge can engage one or more mating recesses 360 in the base (*see* Fig. 4a). In an embodiment, such recesses 360 can include an interference lock such as a spring detent or other locking means. In an embodiment, such other locking means can include a physical interference that can require affirmative/positive action by the user to release the cartridge. Such action can include or require actuation of a release lever 362. Additionally, the cartridge can include one or more locator pins (not shown) configured to passively fit into mating holes on the base (*e.g.,* 364 in Fig. 4a).

In operation, a user first manually positions catheter 406 and sheath 410 (with catheter 406 inserted in sheath 410) within the vasculature of a patient. Once the medical devices are roughly positioned in relation to the heart or other anatomical site of interest, the user can then engage or connect (*e.g.,* "snap-in") the catheter and sheath cartridges into place on respective bases 308, 310. When a cartridge is interconnected with a base, the fingers fit into the recesses formed in the slider blocks. For example, with respect to the sheath cartridge 404 and sheath base 310, each of the plurality of fingers 316, 318, 320 or 322 fit into corresponding recesses formed between the distal edge of slider blocks 412, 414, 416, 418 and a lower portion of the cartridge housing (best shown in Fig. 5b). Each finger can be designed to be actuated in a proximal direction to respectively move each slider block, thereby placing the respective steering wire in tension (*i.e.,* a "pull" wire). Translation, distal end bending and virtual rotation can be accomplished through the use of the RCGS 10.

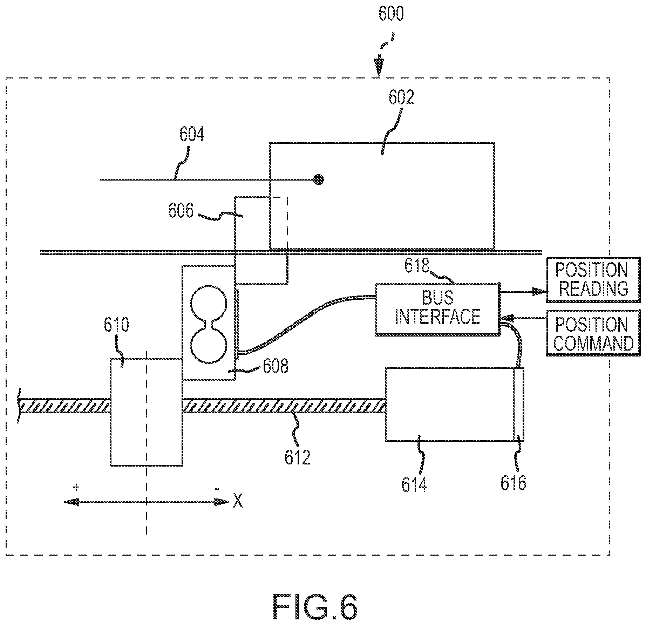

Figure 6 is a diagrammatic view of a node suitable for connection to a communications bus (not shown) in RCGS 10. The node includes an actuation unit 600, similar to the actuation mechanisms described above (*e.g.,* catheter actuation mechanism 304). The RCGS 10 can have at least ten such actuation units (*i.e.,* one for each of the four catheter steering wires, four sheath steering wires, one catheter manipulation base and one sheath manipulation base), which as described include electric motors. The diagnostic logic of the present disclosure is configured to monitor all the electric motors to detect runaway motor fault conditions.

Figure 6 shows in diagrammatic or block form many of the components described above-where appropriate, references to the earlier describe components will be made. Actuation unit 600 includes a first, slidable control member 602 (*i.e.,* slider as described above) that is connected to or coupled with a second, tensile control member 604 (*i.e.,* steering wire as described above). The slider 602 can be configured to interface with a third, movable control member 606 (*i.e.,* finger as described above). The finger 606 can further be operatively coupled with a portion of a sensor 608 (*e.g.,* a force sensor), which, in turn, can be coupled with a translatable drive element 610 that can be mechanically moved. For example, without limitation, translatable drive element 610 can ride on or can otherwise be mechanically moved by a mechanical movement device 612 that, in turn, can be coupled with an electric motor 614. The mechanical movement device 612 can comprise a lead screw while the translatable drive element 610 can comprise a threaded nut, which can be controllably translated by screw 612 in the X+ or X- directions. In another embodiment, mechanical movement device 612 can include a ball screw, while translatable drive element 610 can include a ball assembly. Many variations are possible, as will be appreciated by one of ordinary skill in the art.

The actuation unit 600 also includes a rotary motor position encoder 616 that is coupled to the motor 614 and is configured to output a signal indicative of the position of the motor 614. The encoder 616 can comprise an internal, optical encoder assembly, integral with motor 614, configured to produce a relatively high accuracy output. The motor position sensor can operate in either absolute or relative coordinates. In an embodiment, a second motor position sensor (not shown) can also be provided, such as a potentiometer (or impedance-based), configured to provide a varying voltage output proportional to the motor's rotary position. The output of the secondary position sensor can be used as an integrity check of the operating performance of the primary position sensor (encoder) during start-up or initialization of the actuation unit.

Actuation unit 600 also includes one or more local controllers including a bus interface 618 to facilitate exchange of information between actuation unit 600 and electronic control system 200 (via the bus). The controller communicates with the main electronic control system 200 via the bus interface and is configured, among other things, to (1) receive and execute motor actuation commands issued by the electronic control system 200 for controlling the movements of motor 614; and (2) receive and execute a command (issued by the electronic control system 200) to take a motor position sensor reading, for example, from encoder 616 and subsequently report the reading to system 200.

*Proximity/Contact Sensing.* A proximity/contact sensor on the catheter provides a proximity signal indicating how close (*i.e.,* distance) the catheter is to the nearest body tissue (*e.g.,* heart tissue). Embodiments are configured to monitor this proximity/contact signal periodically, *e.g.,* every input/output cycle of the RCGS, and take appropriate response action, to ensure that the location of the catheter is always kept at a prudently safe distance from any unexpected anatomical structures, so as to avoid catheter-to-tissue contact at speed. Embodiments are configured to automatically implement such response actions, such as to alter (*i.e.,* reduce) device speed or alternatively to cut power to the RCGS motors.

A variety of proximity/contact sensors can be used, for example, an optical force sensor or a mechanical force sensor, either being suitable for determining proximity/contact. For example, one such optical force sensor can be found in an ablation catheter commercially available under the trade designation TactiCath from Endosense, Geneva, Switzerland. In one embodiment, a so-called electrical coupling index (ECI), or a derivative thereof such as a rate of change of ECI, can be used to indicate proximity of an electrode on the catheter to the nearest tissue. The ECI is a figure of merit derived or otherwise computed using, among other things, components of the measured complex impedance between the catheter electrode and the body tissue. The relationship between ECI and components of the complex impedance can be determined empirically. A brief description of such a process will be set forth below.

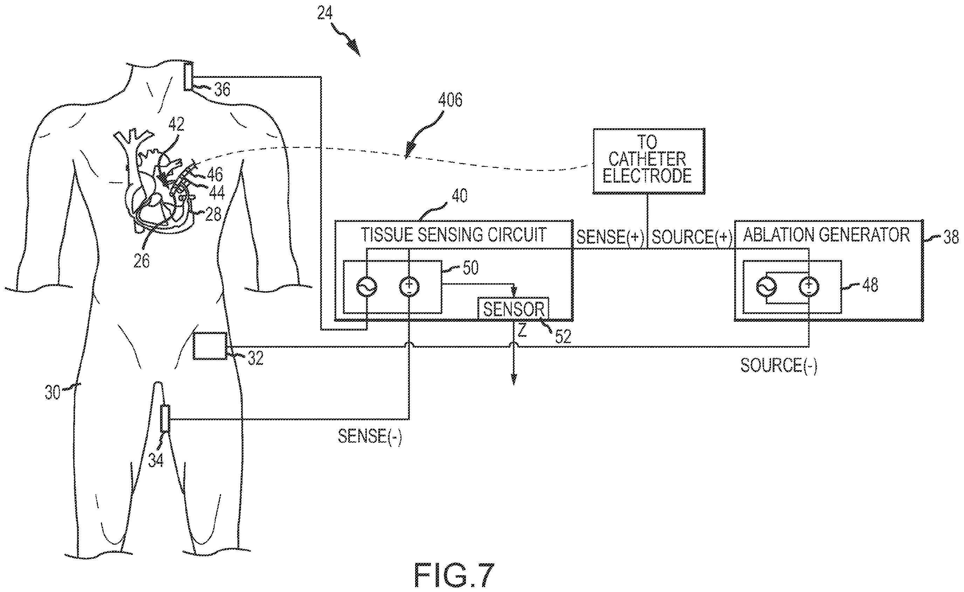

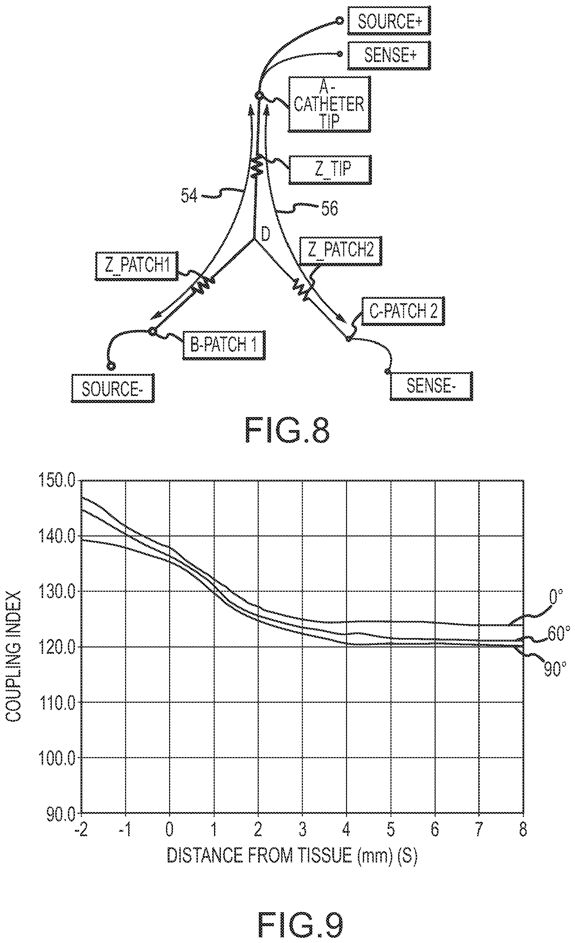

Figure 7 is a diagrammatic and block diagram of the setup of an exemplary system 24 that can be used for determining a degree of electrical coupling between an electrode 26 on a catheter (*e.g.,* catheter 406) and a tissue 28 in a body 30. The degree of coupling can be useful for assessing, among other things, the degree of contact between the electrode 26 and the tissue 28, as well as the relative proximity of the electrode 26 to the tissue 28. In addition to the catheter 406, the system 24 can include patch electrodes 32, 34, 36, an ablation generator 38, and a tissue sensing circuit 40. The catheter 406 has a proximal end and a distal end 42 and one or more electrodes 26 (tip), 44 (ring), 46 (ring). The patch electrode 32 can function as an RF indifferent/dispersive return for an RF ablation signal. The patch electrodes 34, 36 can function as returns for either the RF ablation signal source and/or an excitation signal generated by the tissue sensing circuit 40. Ablation generator 38 includes an RF source 48 while tissue sensing circuit 40 includes an excitation source 50. Circuit 40 also includes an impedance sensor 52. While ablation generator 38 is shown, it should be understood that its presence is not necessary for determining ECI. The SOURCE (+), SOURCE (-), SENSE (+) and SENSE (-) connectors shown correspond to that shown in Figure 8.

Referring now to Figure 8, connectors SOURCE (+), SOURCE (-), SENSE (+) and SENSE (-) form a three terminal arrangement permitting measurement of the complex impedance at the interface of the tip electrode 26 and the tissue 28. Complex impedance can be expressed in rectangular coordinates as set forth in equation (1):

$$(1) \qquad Z = R + jX$$

where R is the resistance component (expressed in ohms); and X is a reactance component (also expressed in ohms). Complex impedance can also be expressed through polar coordinates as set forth in equation (2):

$$(2) \qquad Z = r \cdot e^{j\theta} = |Z| \cdot e^{j\angle Z}$$

where $|Z|$ is the magnitude of the complex impedance (expressed in ohms) and $\angle Z = \theta$ is the phase angle expressed in radians. Alternatively, the phase angle can be expressed in terms of degrees where $\phi = \left(\dfrac{180}{\pi}\right)\theta$. Phase angle will be preferably referenced in terms of degrees. The three terminals comprise: (1) a first terminal designated "A-Catheter Tip" which is the tip electrode 26; (2) a second terminal designated "B-Patch 1" such as the source return patch electrode 36; and (3) a third terminal designated "C-Patch 2" such as the sense return patch electrode 34. In addition to the ablation (power) signal generated by the source of the ablation generator 38, an excitation signal generated by the source 50 in the tissue sensing circuit 40 is also being applied across the source connectors (SOURCE (+), SOURCE(-)) for the purpose of inducing a response signal with respect to the load that can be measured and which depends on the complex impedance. In one embodiment, a 20 kHz, 100 μA AC constant current signal is sourced along a path 54, as illustrated, from one connector (SOURCE (+), starting at node A) through the common node (node D) to a return patch electrode (SOURCE (-), node B). The complex impedance sensor 52 is coupled to the sense connectors (SENSE (+), SENSE (-)), and is configured to determine the impedance across a path 56. For the constant current excitation signal of a linear circuit, the impedance will be proportional to the observed voltage developed across SENSE (+)/SENSE(-), in accordance with Ohm's Law: Z=V/I. Because voltage sensing is nearly ideal, the current flows through the path 54 only, so the current through the path 56 (node D to node C) due to the excitation signal is effectively zero. Accordingly, when measuring the voltage along the path 56, the only voltage observed will be where the two paths intersect (*i.e.,* from node A to node D). Depending on the degree of separation of the two patch electrodes (*i.e.,* those forming nodes B and C), an increasing focus will be placed on the tissue volume nearest the tip electrode 26.

An ECU (not shown in Fig. 7, but can be part of electronic control system 200-Fig. 1) can be provided to acquire values for first and second components of a complex impedance (*i.e.,* the resistance (R) and reactance (X) or the impedance magnitude ($|Z|$) and phase angle ($\phi$) or any combination of the foregoing or derivatives or functional equivalents thereof) between the catheter tip electrode 26 and the tissue 28 and to calculate an ECI responsive to the values with the coupling index indicative of a degree of coupling between the electrode 26 and the tissue 28.

The ECI can be computed using an equation (*e.g.,* equation 3 below) but particular coefficients, or in other words, the relationship to the measured complex impedance components can vary depending on, among other things, the specific catheter used, the patient, the equipment, the desired level of predictability, the species being treated, and disease states.

Validation testing relating to the coupling index was performed in a pre-clinical animal study. The calculated coupling index was compared to pacing threshold as an approximation of the degree of coupling. Pacing threshold was used for comparison because it is objective and particularly sensitive to the degree of physical contact between the tip electrode and tissue when the contact forces are low and the current density paced into the myocardium varies. In a study of seven swine (n=7, 59 +/- 3 kg), a 4 mm tip irrigated RF ablation catheter was controlled by an experienced clinician who scored left and right atrial contact at four levels (none, light, moderate and firm) based on clinician sense, electrogram signals, three-dimensional mapping, and fluoroscopic images. Several hundred pacing threshold data points were obtained along with complex impedance data, electrogram amplitudes and data relating to clinician sense regarding contact. A regression analysis was performed using software sold under the registered trademark "MINITAB" by Minitab, Inc. using the Log10 of the pacing threshold as the response and various impedance parameters as the predictor. The following table summarizes the results of the analysis:

| Model | Regression Factors in Model | | | | | Regression R^2 | |
|---|---|---|---|---|---|---|---|
| | | | | | | R^2 | R^2_adj |
| 1 | | | | | R1_mean (p<0.001) | 43.60% | 43.50% |
| 2 | | | | | X1_mean (p<0.001) | 35.70% | 35.50% |
| 3 | | | | X1_mean (p<0.001) | R1_mean (p<0.001) | 47.20% | 46.90% |
| 4 | | X1_stdev (p=0.300) | R1_stdev (p=0.155) | X1_mean (p<0.001) | R1_mean (p<0.001) | 48.70% | 48.00% |
| 5 | R1_P-P (p=0.253) | X1_stdev (p=0.280) | R1_stdev (p=0.503) | X1_mean (p<0.001) | R1_mean (p<0.001) | 49.00% | 48.10% |

As shown in the table, it was determined that a mean value for resistance accounted for 43.5% of the variation in pacing threshold while a mean value for reactance accounted for 35.5% of the variation in pacing threshold. Combining the mean resistance and mean reactance values increased the predictive power to 46.90% demonstrating that an ECI based on both components of the complex impedance will yield improved assessment of coupling between the catheter electrode 26 and the tissue 28. As used herein, the "mean value" for the resistance or reactance can refer to the average of N samples of a discrete time signal $x_i$ or a low-pass filtered value of a continuous $x(t)$ or discrete $x(t_i)$ time signal. As shown in the table, adding more complex impedance parameters such as standard deviation and peak to peak magnitudes can increase the predictive power of the ECI. As used herein, the "standard deviation" for the resistance or reactance can refer to the standard deviation, or equivalently root mean square about the mean or average of N samples of a discrete time signal $x_i$ or the square root of a low pass filtered value of a squared high pass filtered continuous $x(t)$ or discrete $x(t_i)$ time signal. The "peak to peak magnitude" for the resistance or reactance can refer to the range of the values over the previous N samples of the discrete time signal $x_i$ or the $k^{th}$ root of a continuous time signal $[abs(x(t))]^k$ that has been low pass filtered for sufficiently large $k > 2$. It was further determined that, while clinician sense also accounted for significant variation in pacing threshold (48.7%)--and thus provided a good measure for assessing coupling--the combination of the ECI with clinician sense further improved assessment of coupling (accounting for 56.8% of pacing threshold variation).

Because of the processing and resource requirements for more complex parameters such as standard deviation and peak to peak magnitude, and because of the limited statistical improvement these parameters provided, it was determined that the most computationally efficient ECI would be based on mean values of the resistance (R) and reactance (X), and more specifically, the equation: ECI = a*Rmean + b*Xmean + c.

From the regression equation, and using a 4 mm irrigated tip catheter, the best prediction of pacing threshold-and therefore coupling-was determined to be the following equation (3):

$$(3) \quad ECI = Rmean - 5.1 * Xmean$$

where Rmean is the mean value of a plurality of resistance values and Xmean is the mean value of a plurality of reactance values. It should be understood, however, that other values associated with the impedance components, such as a standard deviation of a component or peak to peak magnitude of a component which reflect variation of impedance with cardiac motion or ventilation, can also serve as useful factors in the ECI. Further, although the above equation and following discussion focus on the rectangular coordinates of resistance (R) and reactance (X), it should be understood that the ECI could also be based on values associated with the polar coordinates impedance magnitude ($|Z|$) and phase angle ($\phi$) or indeed any combination of the foregoing components of the complex impedance and derivatives or functional equivalents thereof. Finally, it should be understood that coefficients, offsets and values within the equation for the ECI can vary depending on, among other things, the specific catheter used, the patient, the equipment, the desired level of predictability, the species being treated, and disease states. However, the coupling index will always be responsive to both components of the complex impedance in order to arrive at an optimal assessment of coupling between the catheter electrode 26 and the tissue 28.

The above-described analysis was performed using a linear regression model wherein the mean value, standard deviation, and/or peak to peak magnitude of components of the complex impedance were regressed against pacing threshold values to enable determination of an optimal ECI. It should be understood, however, that other models and factors could be used. For example, a nonlinear regression model can be used in addition to, or as an alternative to, the linear regression model. Further, other independent measures of tissue coupling such as atrial electrograms could be used in addition to, or as an alternative to, pacing thresholds.

Figure 9 illustrates examples of the results of ECI calculations that are meant to correspond to calculations representing three different angles of approach - 0, 60, and 90 degrees - of the electrode 26 to the tissue 28. As shown, the ECI increases as the distance between the catheter tip electrode 26 and the tissue 28 decreases. A detailed description of an exemplary approach of calculating the ECI and assessing the degree of contact is set forth in U.S. Patent Application No. 12/095,688, filed May 30, 2008 (U.S. Publication No. 2009/0163904) entitled "SYSTEM AND METHOD FOR ASSESSING COUPLING BETWEEN AN ELECTRODE AND TISSUE". A detailed description of another exemplary approach or technique for determining proximity based on ECI is set forth in U.S. Patent Application No. 12/465,337 filed May 13, 2009 (U.S. Publication No. 2009/0275827) entitled "SYSTEM AND METHOD FOR ASSESSING PROXIMITY OF AN ELECTRODE TO TISSUE IN A BODY".

*Proximity/Contact Sensor Interface in the RCGS.* An apparatus for use in the RCGS 10 as described herein minimizes or eliminates unintended contact of a robotically-controlled medical device (*e.g.*, catheter), thereby reducing or eliminating unintended tissue trauma (*e.g.*, perforation). Embodiments of the disclosure establish a plurality of so-called proximity zones. Each proximity zone can have an associated set of proximity criteria which, if met by the position, speed and deflection/rotation of the medical device being monitored, results in predetermined response actions being taken. Embodiments are configured to obtain specifications from the user to establish multiple proximity zones (*e.g.*, "GREEN", "YELLOW" and "RED" proximity zones, A-Z proximity zones, 1-10 proximity zones, etc.). For example, the RCGS 10 can guide the catheter in the patient's body at "normal" speeds (*i.e.*, a default speed) while in the GREEN proximity zone, since the GREEN proximity zone is sufficiently spaced from nearby anatomical structures. However, the RCGS 10 can guide the catheter at only a reduced speed while in the YELLOW proximity zone. This action is based on the premise that the catheter has moved "close enough" to an anatomical structure, based on a proximity signal, to trigger a more cautious, reduced speed. When the catheter enters the RED proximity zone, embodiments of the apparatus will terminate power to the actuation mechanisms (*i.e.*, motors) in the RCGS to prevent unintended catheter-to-tissue contact. Termination of power is based on the apparatus' determination, based on the proximity signal, that the catheter is "too close" to the anatomical structure and immediate action must be taken to avoid contact. In a related embodiment, when the proximity signal crosses a threshold, or nears a threshold, the control signal to the actuation mechanism(s) of the RCGS 10 can temporarily reverse or return to a prior location known to be free of obstacles or undesired contact with anatomical structure(s).

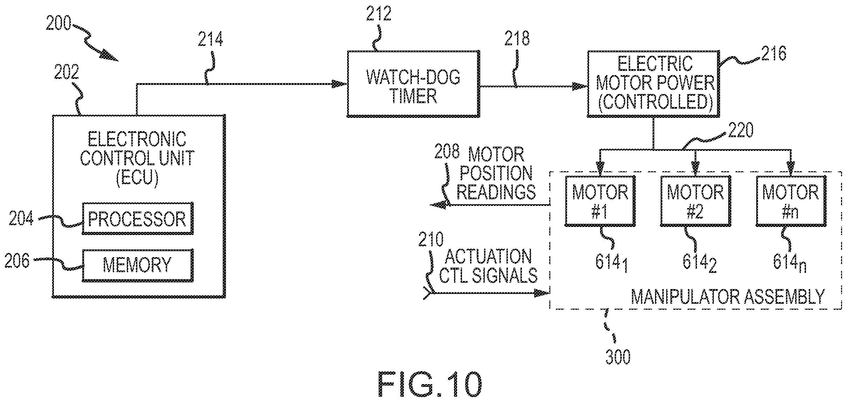

Figure 10 is a block diagram showing electronic control system 200 of Figure 1, in which embodiments of the proximity sensor interface can be implemented. The system 200 includes an electronic control unit (ECU) 202 having a processor 204 and an associated computer-readable memory structure 206. The system 200 further includes logic, which in an embodiment can take the form of software stored in memory 206 and configured for execution by the processor 204. The ECU 202 can comprise conventional apparatus known in the art. Generally, the ECU 202 is configured to perform not only the proximity sensor interface functions described herein, but also the core operating functions of the RCGS 10. As to the latter, the ECU 202 is configured to interpret user inputs, device location data, motor position readings 208 as well as other inputs and generate a plurality of actuation control signals 210, which are provided to the manipulator assembly 300. The actuation control signals 210 in turn are configured to control the plurality of electric motors $614_1$, $614_2$, ..., $614_n$ so as to actuate a plurality of control members of the medical device (*e.g.*, pull wires for deflection movement, manipulation bases for translation movement). While the exemplary RCGS 10 includes robotic control mechanisms for guiding the movement of both a catheter and a sheath, embodiments can be used in connection with a wide range of medical devices, in addition to a catheter and sheath. However, for ease of description, the medical device will be referred to as a catheter.

As described above, when the approach of the catheter relative to a body structure (*e.g.*, cardiac wall) meets the most serious proximity criteria (*i.e.* RED proximity zone), the ECU 202 will terminate operating power to the electric motors $614_1$, $614_2$, ... , $614_n$ as a safety precaution (*i.e.*, to avoid potential tissue damage). To this end, the RCGS 10 can include a mechanism for selectively terminating operating power, for example only, a mechanism that includes a watchdog timer 212 or the like. The watchdog timer 212 is configured to have a countdown time interval which counts down (or up if so configured). The timer 212, which is coupled to a controlled power source 216, is configured to automatically generate a power termination signal 218 when the countdown time interval expires. The power source 216, in response to the power termination signal 214, will terminate operating power 220 provided to the motors. While the timer 212 and the power source 216 are shown separately, they can be integrated into a single unit.

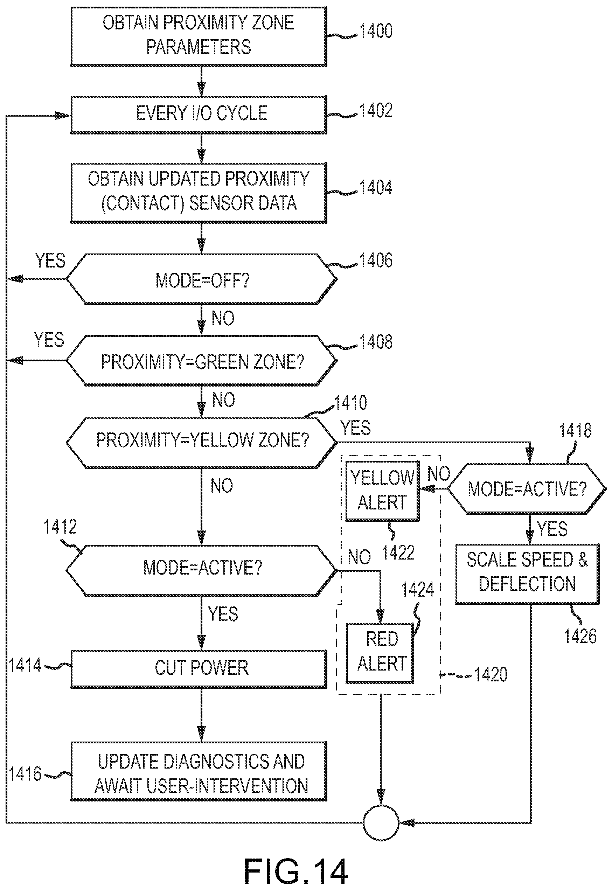

To prevent the watchdog timer 212 from terminating power during normal operation, the ECU 202 is programmed (*e.g.*, in an operating control routine) to generate, at times less than the countdown time interval, a set (or reset) signal 214 in order to refresh the countdown time interval. For example, this refresh can occur every I/O cycle of the RCGS 10. The I/O is described below in connection with Figure 14. Thus, the watchdog timer 212 will automatically shut off the power unless the ECU specifically confirms (*e.g.*, through the periodic assertion of the refresh signal 214) that it is operating normally. However, when the catheter has moved into the RED proximity zone, the ECU 202 suppresses the refresh signal 214, thereby allowing the watchdog timer to expire, in turn automatically terminating operating power to the motors, stopping the catheter. It should be understood that other mechanisms, including software, hardware, or combinations thereof, can by employed to terminate power to the electric motors.

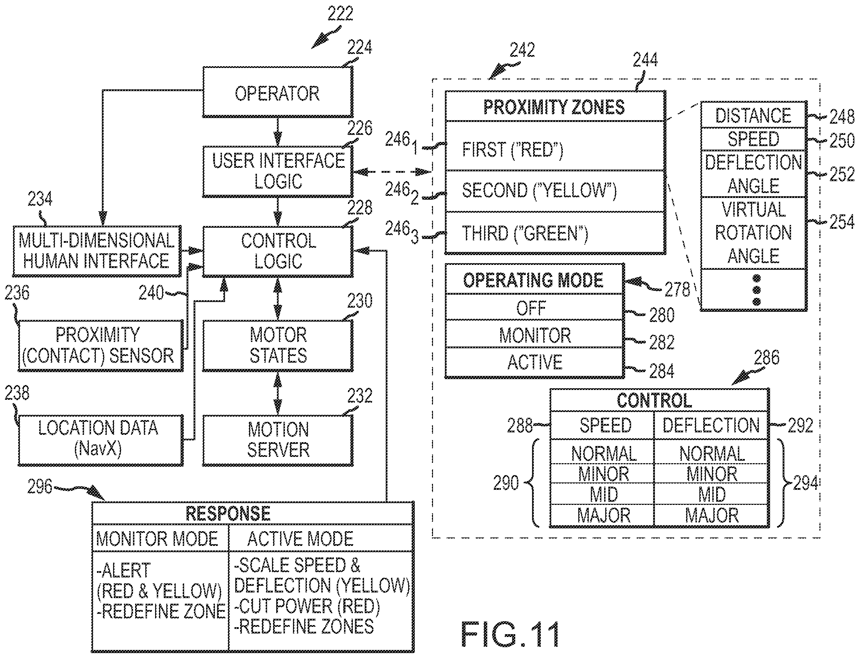

Figure 11 is block diagram of an apparatus 222, which shows a functional configuration of the electronic control system 200 in which the proximity sensor interface is implemented. The apparatus 222 is configured for interaction with an operator/user 224. In many instances, the user 224 can be an electrophysiologist (EP) or the like that is manipulating the catheter via the RCGS 10. The apparatus 222 includes user interface (UI) logic 226, operating control logic 228, a motor state model 230, and a motion server 232. The apparatus 222 receives a variety of inputs from the user 224 via UI logic 226 including inputs relating to the proximity sensor interface, as described in detail below. In addition, the operator 224 can manipulate the multi-dimensional controller 234, which is part of input control system 100, as another means of providing inputs (*e.g.*, inputting desired catheter motions, rotating an anatomical model on a workstation display, etc.). Figure 11 also shows a proximity sensor 236 and a source of localization data 238.

The UI logic 226 performs the general functions of outputting information (visual, textual, aural, etc.) for the user as well as querying for or otherwise permitting entry of inputs from the user 224. This general function applies to both core operations of the RCGS 10 as well as for the proximity sensor interface. For example, the UI logic 226 displays information regarding a currently displayed (rendered) scene (*e.g.,* the view angle, the mouse location, the catheter tip location, etc.). The UI logic 226 is also configured to receive user inputs with respect to an anatomical model of a body portion of the patient, for example, for setting up a pre-planned path for the catheter, for initiating and conducting diagnostic and therapeutic procedures, or the like. With respect to the proximity sensor interface, the UI logic 226 receives inputs from the user 224 to define the plurality of proximity zones (*e.g.*, distance, speed, etc.), an operating mode of the proximity sensor interface (*e.g.,* OFF, MONITOR, ACTIVE, etc.), control actions (*e.g.,* speed reductions) and response actions (*e.g.*, alerts, terminating power, etc.).

The control logic 228 is configured, generally, to implement a predetermined operating control strategy (*i.e.,* higher level control algorithms) for the RCGS 10, as described in greater detail in U.S. application no. 12/751,843 filed 3/31/2010 entitled "ROBOTIC CATHETER SYSTEM" that was referred to above. The operating control logic 228 is configured to process incoming data from a plurality of sources, including the UI logic 226, the human interface device 234, a proximity signal 240 from the proximity sensor 236, location data 238, as well as the current motor states from the motor state model 230. Based on these inputs, the apparatus 222 generates actuation control signals 218 destined for the plurality of motors in the manipulator assembly to achieve desired catheter and sheath movements (*i.e.,* translation, deflection or virtual rotation).

The human interface device 234 facilitates communication of inputs by the user 224 (*i.e.,* directions) to the apparatus 222. For example, for a controller type device, the control logic 228 can receive a current handle position as well as current button states for those devices having buttons. For example only, the handle location can be specified in a frame of reference (*i.e.,* a 2-dimensional or 3-dimensional coordinate system) that is specific to the device (*e.g.*, x, y, z).

In an embodiment, the proximity signal 240 can take the form of an ECI as described above in connection with Figures 7-9, or a derivative quantity thereof (*e.g.,* a first or higher order time derivative of ECI). It should be emphasized that the proximity signal 240 is not merely the position of the catheter as provided by the localization system, but is in fact a signal indicative of the proximity (distance, nearness) of the catheter to an anatomical structure, such as a cardiac wall or other body tissue.

Location data from source 238 can comprise position and orientation information associated with the manipulated catheter (*e.g.*, such as the catheter tip). In an embodiment where an impedance-based positioning system (*e.g.*, ENSITE VELOCITY) 14 is used as the source 238, the location data can comprise at least an electrode coordinate (x, y, z) for specified electrodes on the catheter.

The motor state model 230 contains information about the current states for each of the motors in the RCGS 10 (*i.e.,* reflects the current physical states of the physical motors). States can include motor position, motor speed, tension (*i.e.,* pull wire tension-see Figure 6). The motion server 232 is configured to interpret movement commands in a way so as to achieve the motor states specified in the motor state model 230 (*e.g.,* an actuation command to a motor to achieve a desired motor position). The motor server 232 also communicates information to the model 230 that describes the current physical states of the motors in the RCGS 10 (*e.g.,* a motor encoder reading indicative of a motor position).

An exemplary use case involves the user 224 specifying a number of so-called waypoints to describe a movement path having a predetermined, default or specified speed (or speed profile along the path) for the catheter. The pre-planned movement is then executed by the RCGS 10. However, the catheter, as guided by the RCGS 10 in accordance with the pre-planned movement, can unexpectedly encounter an anatomical structure. The apparatus 222 is configured to monitor proximity, based on the proximity signal 240, speed, and deflection/rotation, throughout the catheter's pre-planned movement. When predetermined proximity criteria are met, the apparatus 222 selectively adjusts the pre-planned movement consistent with the likelihood of a potential contact, for example, by either reducing speed or terminating power to the motors, thereby stopping the catheter before completion of the pre-planned path.

The apparatus 222 uses a construct referred to herein as a proximity zone, a descriptor that is used to refer to a number of individual pieces of information that collectively define proximity criteria for each zone. Each proximity zone also has an associated set of response actions, which are fulfilled by the apparatus 222 when the catheter's movement in relation to any nearby tissue meets the criteria for that proximity zone. To setup the proximity zone, the user 224 interacts with the UI logic 226 to obtain the needed data. Several categories of such data are thus provided by the user 224 and are collectively shown in block 242 in Figure 11.

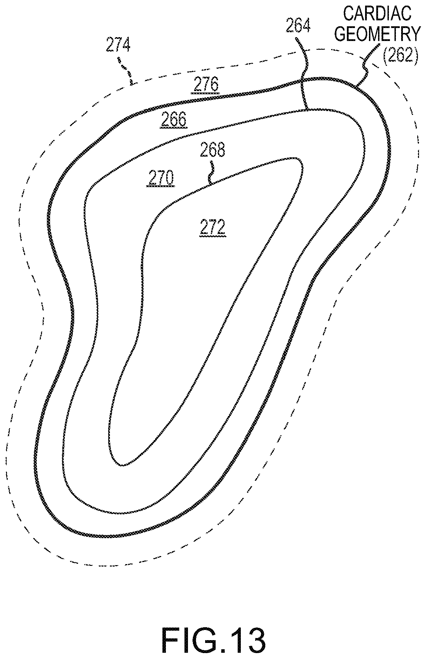

A proximity zone table 244 includes a plurality of records $246_1$, $246_2$, $246_3$ identifying the criteria associated with each of a plurality of proximity zones (*i.e.,* a physical representation of multiple proximity zones is shown in Figure 13). In the illustrated embodiment, the first record $246_1$ in the table 244 corresponds to a first proximity zone (hereinafter also referred to herein as the RED proximity zone), which is the most restrictive proximity zone in the RCGS 10. The second record $246_2$ in the table 244 corresponds to a second proximity zone (hereinafter also referred to herein as the YELLOW proximity zone), which is less restrictive than the first proximity zone but nonetheless warrants a response action (*e.g.,* a reduced catheter speed). The third record $246_3$ of the table 244 corresponds to the third proximity zone (hereinafter also referred to herein as the GREEN proximity zone), which is the least restrictive and where no additional adjustments to the pre-planned movement of the catheter are made. Although three proximity zones are described by way of example, the apparatus 222, in other embodiments, can be configured to provide fewer or greater number of proximity zones.

Each record $246_i$ (where i=1 to *n*-the number of proximity zones) has a respective plurality of fields associated therewith, such as a proximity-to-tissue distance 248, a device speed 250 (user-specified), and a deflection/rotation, for example, which can include a deflection angle 252 and a virtual rotation angle 254. The UI logic 226 is configured to receive inputs from the user 224 to set values for some of these fields, depending on the embodiment.

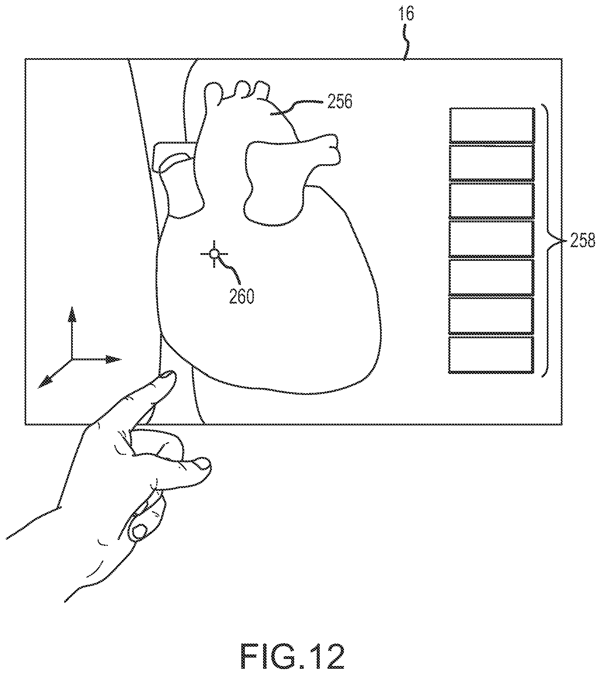

Figure 12 is a depiction of an anatomical model 256, as shown on monitor 16, which can represent a region of interest within the patient's anatomy (*e.g.,* the patient's heart). In the illustrated embodiment, the model 256 reflects the geometry of the subject anatomical structure, and can be of the type produced by a visualization, mapping and navigation system 14 (*i.e.,* ENSITE VELOCITY). It should be understood, however, that other sources can provide suitable representations of the subject anatomical structure, such as imaging data obtained through the use of computed tomography or magnetic resonance imaging systems. The monitor can display a variety of other information, such as the current location of the catheter tip, shown at point 260. Generally, the UI logic 226 receives user inputs to direct execution of a variety of functions, including, for example only, panning, rotating, or zooming 3D objects and models within the display, selecting and/or directing movement of the catheter or sheath, placing lesion markers, way points (*i.e.,* as described above in order to specify a pre-planned movement for the catheter), virtual sensors, or automated movement targets and lines on or within the anatomic model 256. The UI logic 226 provides, in an embodiment, a graphical mechanism to receive such inputs, such as by providing on-screen menu buttons 258 or the like with which the user 224 interacts in order to make selections or otherwise provide inputs (*e.g.,* specifying values, selections between multiple options, etc.). The user 224 can interact with UI logic 226 through conventional means (*e.g.,* mouse, keyboard, touch screen, etc.). The user 224 interacts with the apparatus 222 via UI logic 226 to specify and setup the proximity zones, as described below in connection with Figure 13.

Figure 13 is a simplified two-dimensional diagrammatic view of a plurality of proximity zones defined in a patient's anatomy. The UI logic 226 displays the anatomical model 256 or other representation as a frame of reference for the user 224, and with respect to which the user 224 can specify the required data needed to set up the proximity zones. The frame of reference need not be anatomically accurate for this purpose or phase of the set-up. The frame of reference in Figure 13 shows a closed-line boundary 262 that identifies a surface representing the tissue of the anatomical structure, referred to herein as a tissue boundary 262. In the displayed frame of reference, the catheter or other medical device to be navigated by the RCGS 10 resides in the cavity that is bounded by the tissue boundary 262 and which extends inwardly of the boundary 262.

In an embodiment, the apparatus 222 is pre-configured with a predetermined number of proximity zones (*e.g.*, three), each of which are pre-defined in terms of a respective distance-from-tissue value (*i.e.,* field 248) as well as with respect to the response action to be taken when the respective criteria are met (*e.g.*, reduce speed in the Yellow zone, cut power in the Red zone). In other words, in this embodiment, while the number of zones, the distances and response actions are configurable (*i.e.,* at the design time of the RCGS 10), they are not *user* configurable through UI logic 226. Additionally, however, in this embodiment, the user 224 can specify other criteria associated with the proximity zones (*e.g.,* catheter speed).

In another embodiment, however, the UI logic 226 is configured to receive inputs to set the number of zones, the respective characteristics or criteria (*i.e.,* the distance 248, speed 250, deflection angle 252, virtual rotation parameter 254), control actions (below) and response actions (below). In a further embodiment, the apparatus 222 is configured to use a combination of pre-configured and user-specified values (obtained through UI logic 226).

With continued reference to Figure 13, the control logic 228 of the apparatus 222 sets a first boundary 264 at a first distance from the tissue boundary 262 to establish a first proximity zone 266 (corresponding to the first record $246_1$-the RED proximity zone). The first zone 266 is thus between the first boundary 264 and the tissue boundary 262. Likewise, the apparatus 222 sets a second boundary 268 at a second distance from the tissue boundary 262 to establish a second proximity zone 270 (corresponding to the second record $246_2$-the YELLOW proximity zone). The second zone 270 is thus between the second boundary 268 and the first boundary 264. As shown, the second distance is greater than the first distance. The control logic 228 establishes a third proximity zone 272 for distances from the tissue boundary 262 greater than the second distance (*i.e.,* beyond the second boundary 268). The third proximity zone 272 corresponds to that specified in the third data record $246_3$-the GREEN proximity zone).

The distance-to-tissue, speed threshold, and deflection/rotation values appropriate for any particular RCGS 10 can take a wide range of values. For example, it should be appreciated that mechanical inertia of the components of the RCGS 10 can be considered in selecting distance/speed combinations for the plurality of proximity zones (*i.e.,* a "stopping distance" to avoid contact of a moving catheter with tissue). The deflection angle and/or rotation/virtual rotation value can also come into play as distal end shape affected by these parameters can in turn affect the angle of approach. In any event, for exemplary purposes only, the first proximity zone 266 can be set up to span distances from 0 mm to 2 mm from the tissue boundary 262, the second proximity zone 270 can be set up to span distances greater than 2 mm but less than 4 mm from the tissue boundary 262 and the third proximity zone 272 can be set up to cover distances greater than 4 mm from the tissue boundary 262. Of course, as described, these distances are exemplary only and the actual values will vary. For exemplary purposes only, a "normal" device speed (*e.g.,* in the GREEN proximity zone) within the RCGS 10 can be 5 mm/second, but the particular speed threshold for the YELLOW and RED proximity zones would be reduced, and will vary from system to system. Figure 13 shows a further proximity zone 276 established by a further boundary 274, which is defined as a negative distance from the tissue boundary 262. As shown in Figure 9, an ECI value (*i.e.,* the proximity signal) can continue to be provide a valid assessment of the proximity of the catheter to the tissue boundary 262, even after initial contact between the catheter and the tissue has been made. In a post-initial contact situation, the ECI, in effect, continues to provide a real time assessment of the degree of coupling or contact.

It should be understood that while Figure 13 shows closed boundary proximity zones, which represent closed areas (2D) or volumes (3D), this characteristic is exemplary only and not limiting in nature. The distance proximity criteria applies to all anatomical structures, with respect to which such proximity zones are defined, need not be closed.

Referring again to Figure 11, the UI logic 226 is configured to receive further inputs from the user 224 to specify an operating mode for the proximity sensor interface of the RCGS 10. The operating mode is user selectable and can be stored in a table or other data structure 278. As shown, the operating mode can be an OFF mode 280, a MONITOR mode 282 or an ACTIVE mode 284.

In the OFF mode 280, the apparatus 220 will inhibit reading of proximity sensor 236 or if such sensor is read, its effect on the operation of RCGS 10 will be suppressed. For example, in the case of an ablation procedure, the user 224 can wish to disable the proximity sensor interface feature of the RCGS 10 by selecting the OFF mode 280.

In the MONITOR mode 282, the apparatus 220 will inhibit the specified response action even when proximity criteria are satisfied and a response action (*e.g.*, reduce speed, cut power) would otherwise be taken by apparatus 222. However, when the proximity criteria associated with a particular zone have been met, the apparatus 220 will generate an alert. Such an alert can be an audio alert, a visual alert, a haptic alert, or a pop-up window displaying a warning or error message. In the ACTIVE mode 284, the apparatus 220 will implement the specified response actions, provided the proximity criteria have been met.

As shown in Figure 11, a response action table 296 contains specified response actions for different modes of operation (MONITOR mode 282 or ACTIVE mode 284). When the apparatus 222 is in the MONITOR mode 282 and the criteria associated with the RED or the YELLOW proximity zones are met, then the apparatus 222 will generate an alert, as described above. When the apparatus 222 is in the ACTIVE mode 284, the apparatus 222 (via control logic 228) will scale (reduce) speed and range of deflection/rotation when the criteria for the YELLOW proximity zone is met. The level of speed and deflection/rotation reduction, if any, is specified in the table 286. When the apparatus 222 is in the ACTIVE mode 284, and the criteria for the RED proximity zone is met, then the power to the RCGS motors will be terminated as described above.

A response action associated with a proximity zone can also modify the behavior of the UI logic 226. In an embodiment, certain functions available in the user interface can be disabled once the proximity zones have been established. For example, the UI logic 226 can be modified to prevent the user 224 from specifying a way point, as part of an overall pre-planned catheter movement, in or through a RED proximity zone.

In another embodiment, the response action can also entail (i) a reversal of movement of the medical device, and/or (ii) a return of the medical device to a prior location within a different proximity zone (*e.g.*, return back to a GREEN zone upon entering a YELLOW zone).

The apparatus 222 is also configured with adaptive logic to dynamically redefine proximity zones. In both the MONITOR mode 282 and the ACTIVE mode 284, the apparatus 222 keeps track of the number of times the catheter entered either the Yellow or Red proximity zones. Using this information, adaptive diagnostic logic (not shown) automatically redefines parameters associated with the proximity zones (*e.g.*, adjust the distance parameter, increase the degree to which the catheter's speed is reduced, etc.). These adjustments are made in a way so as to reduce or eliminate instances of operating power to the motors being terminated.

The control table 286 stores information specifying how and to what extent catheter speed (column 288) or a range of catheter deflection and/or translation (column 292) can be scaled down when the catheter enters the YELLOW proximity zone. The available speed control steps for scaling speed, NORMAL, MINOR, MID, and MAJOR are referred to collectively as steps 290. These steps are selected by the user 224 via interaction through the UI logic 226. The NORMAL step can be the default, normal speed for catheter movement within the RCGS 10, for example only, 5 mm/second. The MINOR step can correspond to a minor reduction, for example, a 25% reduction relative to normal speed. The MID step can correspond to a mid-level reduction, for example, a 50% reduction relative to the normal speed. The MAJOR step can correspond to a large reduction, for example a 75% reduction, relative to normal speed. The speed control step parameters can be selected by the user 224 as the desired response action associated with the YELLOW proximity zone. Likewise, the NORMAL, MINOR, MID, and MAJOR steps associated with deflection step control parameter and translation step control parameters (not shown) can be configured and user selected in a like manner. In another embodiment, the control table 286 may include information to specify a relaxation control step. The relaxation step specifies an amount that any tension force or compression force imparted to the medical device is reduced. For example, the reduction may be one of either wholly released or partially released.

Figure 14 is a flowchart showing a method of operating the RCGS 10, for example, to manipulate a medical device toward a target, using the predefined proximity zones in accordance with a proximity signal. In an embodiment, the proximity signal may be at least one of proximity metric (*e.g.*, ECI) and a contact metric related to the presently detected (*i.e.*, real time) location of the medical device relative to the nearest (or any) nearby tissue. The method begins in step 1400, where the apparatus 222 obtains, via interaction by the user 224 with the UI logic 226, the various thresholds, parameter values and other information described above in connection with Figures 11-13. The method proceeds to step 1402.

In step 1402, the apparatus 222 monitors at predetermined time intervals the catheter's proximity to nearby tissue. In an embodiment, the predetermined time interval can be the I/O cycle time, although other time intervals can be suitable depending on the particular configuration of the RCGS. The RCGS 10 operates in accordance with a system wide input/output (I/O) cycle, which can be on the order of between about 30-50 milliseconds, and can be about 50 milliseconds. The I/O cycle is the "heartbeat" of the RCGS 10, establishing a timing reference for a variety of functions. This deterministic approach ensures that a situation where the catheter unexpectedly approaches an anatomical structure will not go undetected for any more than one I/O cycle's worth of time. The method proceeds to step 1404.

In step 1404, the control logic 228 obtains an updated proximity reading from the proximity sensor 236. As described above, in an embodiment, a value of an ECI can be used, or a derivative of the ECI (*e.g.,* the rate of change of ECI over time). The method proceeds to step 1406.

In step 1406, the control logic 228 determines whether the operating mode has been set to OFF (*e.g.,* by the user, for instance, when conducting ablation where it is desirable to maintain the ablation electrode in good contact with the tissue). If the answer in step 1406 is YES, then no further processing is done and the method branches to step 1402, to await the next I/O cycle. If the answer in step 1406 is NO, then the operating mode is either the MONITOR mode or the ACTIVE mode and in either case, the method proceeds to step 1408.

In step 1408, the control logic 228 determines whether the proximity criteria specified for the GREEN proximity zone has been met. This step can be performed by comparing the defined criteria discussed above to current values for proximity-to-tissue distance, speed, deflection/rotation, etc. For example only, if the distance criterion associated with the GREEN proximity zone specifies that the catheter can be no closer than 4 mm to any anatomical structure (tissue), then the control logic 228 will compare the current proximity reading with a threshold set to correspond to a distance equal to or greater than 4 mm. As shown in Figure 9 for a particular catheter configuration, an ECI of about 120 corresponds to about a distance of about 4 mm from the tissue. Suitable tolerances can be determined and programmed so as to ensure that a proper determination of the current proximity is made. Likewise, the control logic 228 also confirms that the speed and deflection/rotation parameters are also met. If the answer in step 1408 is YES, then the control logic 228 does not need to make any adjustments to the pre-planned movement of the catheter, and accordingly, control of the method branches back to step 1402 to await the next I/O cycle. If the answer in step 1408 is NO, then the method proceeds to step 1410.

In step 1410, the control logic 228 determines whether the proximity criteria is met for the YELLOW (caution) proximity zone. Again, this step can be performed by comparing the defined criteria discussed above to current values for proximity-to-tissue distance, speed, deflection/rotation, etc. If the answer in step 1410 is NO, then the method proceeds to step 1412. Otherwise, if the answer in step 1410 is YES, then the method proceeds to step 1418.

In step 1412, the control logic 228 determines that proximity criteria for the RED proximity zone has been met in the same way as described above for the GREEN and YELLOW proximity zones. The control logic 228, to determine what response action to take, determines whether the operating mode has been set to the ACTIVE mode or to the MONITOR mode. If the control logic 228 determines that the operating mode has been set to the ACTIVE mode, then the method branches to step 1414; otherwise, the method branches to step 1420 (MONITOR mode).

In step 1414 (RED zone, ACTIVE mode), the control logic 228, for example through suppression of the refresh signal 214 (Fig. 10), causes operating power to the electric motors in the manipulator assembly to be terminated, thereby immediately stopping movement of the catheter in accordance with its pre-planned movement. The method then proceeds to step 1416.

In step 1416, the control logic 228 updates an internal register or the like that stores the number of times that power has been terminated (*i.e.*, RED zone). In addition, the control logic 228, given the severity of the response action, is configured to disable further operation of the RCGS 10 until the user 224 specifically intervenes, thereby acknowledging the incident (*e.g.,* a pop-up error message can be displayed through the UI logic 226, and disables further operation of the RCGS 10 until the user clicks to dismiss or otherwise indicates acknowledgement of the error message).

In step 1418, the control logic 228 determines whether the operating mode has been set to the ACTIVE mode. If the answer in step 1418 is NO, then the operating mode has been set to the MONITOR mode, and the method branches to step 1420. Otherwise, the method proceeds to step 1426.

In step 1420 ("MONITOR mode"), the control logic 228 outputs an alert in accordance with the specified alert in the response table 296 (Fig. 11). Step 1420 is shown to include alerts for both a YELLOW zone alert (1422) and a RED zone alert (1424). Both of these alerts can be the same type of alert, or alternatively, the respective alerts can be distinguishable from each other so as to notify the user 224 which proximity zone violation occurred (*i.e.,* either YELLOW or RED). In an embodiment, the control logic 228 is configured to optionally update internal registers or the like that store the respective number of times that that a RED or YELLOW proximity zone violation has been detected. Despite the fact that the operating mode has been set to MONITOR, the diagnostic value of keeping track of the number of detections can still be useful in redefining the proximity zones. The method proceeds to step 1402 to await the next I/O cycle.

In step 1426 (YELLOW zone, ACTIVE mode), the control logic 228 reduces the navigation speed of the catheter (or other device) specified for the pre-planned movement, all in accordance with the speed control (step down) parameters set forth in the control table 286 (Fig. 11). It should be recalled that as an initial matter, the level of speed reduction (MINOR, MID, MAJOR) can be user specified. In addition, the control logic 228 implements the other adjustments, if any, specified in table 286. As described above, in an embodiment, the control logic 228 updates an internal register or the like that keeps track of the number of times the catheter has entered the YELLOW proximity zone. The method then proceeds to step 1402 to await the next I/O cycle.

It should be understood that the sequence of steps in the method of Figure 14 is illustrative only and not limiting in nature. The specific order of steps can be modified, for example, by modifying the order of checking for proximity zone criteria being met (*e.g.,* RED, then YELLOW, then GREEN is an alternative). Further variations are possible.

As described herein, the control logic 228 is configured to keep track of the number of times a reduction in speed was implemented (*i.e.,* YELLOW zone) or that a termination of power was commanded (*i.e.,* RED zone) for the purpose of automatically redefining the proximity zones or response actions associated with a proximity zone. For example, when the number of RED proximity zone entries exceed a predetermined threshold, the control logic 228 can increase a YELLOW proximity zone speed reduction from a MINOR setting to a MAJOR setting. For example, the reason for entry into the RED zone can involve "overshoot" of the catheter into the RED zone from the YELLOW zone due to excessive speed. A reduction in speed in the YELLOW zone can reduce the occurrence of such "overshoot" situations. In another embodiment, the YELLOW zone can be expanded and the RED zone reduced from a dimensional perspective. Other variations are possible.

The proximity sensor interface enhances the experience of the electrophysiologist by providing additional safeguards within the RCGS 10. The capabilities described herein also enhance the safety of the patient by anticipating and avoiding unintended device-to-tissue contact.

While the RCGS 10 as described herein employed linear actuation (*i.e.,* fingers, slider blocks), scope of the disclosures contemplated herein is not so limited and extends to and covers, for example only, a manipulator assembly configured to employ rotary actuation of the control members. In further embodiments, the ECU can be configured to cause the manipulator assembly to either linearly actuate and rotary actuate one or more control members associated with the medical device for at least one of translation, rotation, virtual rotation and deflection movement.

Additional apparatus can be incorporated in or used in connection with the RCGS 10, whether or not illustrated in Figure 1. For example, the following can be coupled (directly or indirectly) to RCGS 10 or used in connection with RCGS 10, depending on the particular procedure: (1) an electrophysiological monitor or display such as an electrogram signal display; (2) one or more body surface electrodes (skin patches) for application onto the body of a patient (*e.g.*, an RF dispersive indifferent electrode/patch for RF ablation); (3) an irrigation fluid source (gravity feed or pump); and (4) an RF ablation generator (*e.g.*, such as a commercially available unit sold under the model number IBI-1500T RF Cardiac Ablation Generator, available from St. Jude Medical, Inc.). To the extent the medical procedure involves tissue ablation (*e.g.*, cardiac tissue ablation), various types of ablation energy sources (*i.e.,* other than radio-frequency-RF energy) can be used in by a catheter manipulated by RCGS 10, such as ultrasound (*e.g.* acoustic/ultrasound or HIFU, laser, microwave, cryogenic, chemical, photo-chemical or other energy used (or combinations and/or hybrids thereof) for performing ablative procedures.

Further configurations, such as balloon-based delivery configurations, can be incorporated into catheter embodiments consistent with the disclosure. Furthermore, various sensing structures can also be included in the catheter, such as temperature sensor(s), force sensors, various localization sensors (see description above), imaging sensors and the like.

As used herein "distal" refers to an end or portion thereof that is advanced to a region of interest within a body (*e.g.,* in the case of a catheter) while "proximal" refers to the end or portion thereof that is opposite of the distal end, and which can be disposed outside of the body and manipulated, for example, automatically through the RCGS 10.

It should be understood that an electronic controller or ECU as described above for certain embodiments can include conventional processing apparatus known in the art, capable of executing pre-programmed instructions stored in an associated memory, all performing in accordance with the functionality described herein. To the extent that the methods described herein are embodied in software, the resulting software can be stored in an associated memory and can also constitute the means for performing such methods. Implementation of certain embodiments, where done so in software, would require no more than routine application of programming skills by one of ordinary skill in the art, in view of the foregoing enabling description. Such an electronic control unit or ECU can further be of the type having both ROM, RAM, a combination of non-volatile and volatile (modifiable) memory so that the software can be stored and yet allow storage and processing of dynamically produced data and/or signals.

It should be further understood that an article of manufacture in accordance with this disclosure includes a computer-readable storage medium having a computer program encoded thereon for implementing the proximity/contact sensor interface described herein. The computer program includes code to perform one or more of the methods disclosed herein.

Although a number of embodiments of this disclosure have been described above with a certain degree of particularity, those skilled in the art could make numerous alterations to the disclosed embodiments without departing from the scope of this disclosure. All directional references (*e.g.*, upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (*e.g.*, attached, coupled, connected, and the like) are to be construed broadly and can include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure can be made without departing from the disclosure as defined in the appended claims.

**Claims**

1. An apparatus for use in a robotic control system for manipulating a medical device toward a target, comprising:

   an electronic control unit (ECU) (202) coupled to a manipulator assembly (300) of the robotic control system and to a proximity sensor (236);
   a computer-readable memory (206) coupled to said ECU (202); and
   control logic (228) stored in said memory (206) configured to be executed by said ECU (202), said control logic (228) configured to produce an actuation control signal (210) to control actuation of a manipulator assembly (300) of the robotic control system so as to navigate the medical device in and with respect to a plurality of pre-defined proximity zones each having a proximity criteria representing a span of distances from from the tissue boundary, said control logic (228) being further configured to generate said actuation control signal (210) based on a proximity signal indicative of at least one of a proximity metric and a contact metric related to a location of the medical device relative to tissue (28) of a patient (30) and in accordance with said pre-defined proximity zones; **characterised in that** said control logic (226) is further configured to generate said actuation control signals so as to navigate said medical device with a reduced deflection determined in accordance with a deflection step control parameter indicative of a decrease in a deflection angle of a distal end of the medical device when the proximity signal meets the criteria of a zone closer to the tissue.

2. The apparatus of claim 1 wherein a pre-planned movement of said medical device includes a pre-planned path and a pre-planned speed profile for the device while moving along said path, said control logic (228) being configured to effect modifications to said pre-planned movement in accordance with said proximity signal and defined characteristics of said proximity zones, wherein said modifications include one of (i) a reduced speed relative to said pre-planned speed profile, (ii) a stoppage of movement of said device before completion of said pre-planned path, (iii) a reversal of movement of said device, and (iv) a return of said device to a prior location within a different proximity zone.

3. The apparatus of claim 1 or 2 further including user interface logic (226) configured to display a view of a representation of a portion of the body (30) and to receive user input with respect to said representation for specifying speed thresholds to be associated with said proximity zones.

4. The apparatus of any one of claims 1 or 3 further including user interface logic (226) configured to receive user input to specify a first distance with respect to said body tissue (28) for determining a first boundary, said proximity zones including a first proximity zone that is established between said first boundary and said body tissue (28).

5. The apparatus of claim 4 wherein said first distance is a characteristic associated with said first proximity zone, said user interface logic (226) is further configured to receive further user input to specify a further characteristic associated with said first proximity zone, including one of a speed threshold and a trajectory, or wherein said user interface logic (226) is further configured to receive further user input to specify a second distance with respect to said body tissue for determining a second boundary, said plurality of proximity zones including a second proximity zone established between said second boundary and said first boundary.

6. The apparatus of claim 5 wherein said plurality of proximity zones includes a third proximity zone beyond said first and second proximity zones.

7. The apparatus of claim 5 wherein said user interface logic (226) is further configured to receive further user input to specify one of (i) a speed step control parameter indicative of a decrease in a navigation speed of the medical device; (ii) said deflection step control parameter indicative of a decrease in a deflection angle of a distal end of the medical device; (iii) a translation step control parameter indicative of a decrease in a translation amount of the medical device, and (iv) a relaxation step wherein any tension forces or compression forces imparted to the medical device is one of wholly released and partially released.

8. The apparatus of claim 1 wherein said plurality of proximity zones include (i) a first zone extending from a tissue boundary through a first distance to a first boundary; (ii) a second zone extending from said first boundary to a second boundary a second distance from said tissue boundary; and (iii) a third zone extending beyond said second boundary;
wherein said control logic is configured to determine, based said proximity signal, in which of said first, second and third zones said medical device reside, said control logic (226) being further configured to generate said actuation control signals so as to,
navigate said medical device normally in accordance with a pre-planned movement having path and speed components when said medical device is in said third zone;
navigate said medical device with a reduced speed relative to said pre-planned speed when said device is in said second zone; and
discontinue navigation of said medical device before completion of the pre-planned path when the medical device is in the first zone.

9. The apparatus of claim 8 wherein said robotic control system includes a manipulator assembly having actuation units configured to actuate one or more control members associated with the medical device in response said actuation control signal; and wherein,
said control logic (226) is configured to cause operating power to said actuation units to be terminated when said medical device is in said first zone.

10. The apparatus of claim 9 wherein said reduction in speed is determined in accordance with a user specified speed step control parameter.

11. The apparatus of claim 8 wherein said control logic is configured to generate an alert when in a user specified monitor mode and said medical device is in one of said first and said second zones.

12. The apparatus of claim 10 wherein said control logic (226) is configured to track the number of times in which said medical device entered one of said first and second zones, said control logic (226) being further configured to increase a value for said speed step control parameter when said number of times exceeds a diagnostic threshold.

13. The apparatus of claim 1 wherein said medical device includes an electrode (26), and wherein said proximity signal is determined as a function of a complex impedance between said electrode (26) and said tissue (28).

14. The apparatus of claim 13 wherein said proximity signal comprises an electrical coupling index (ECI) and/or is a derivative of said ECI.

15. A robotic control and guidance system for manipulating a medical device in a body (28) of a patient (30), comprising the apparatus according to any of claims 1 to 14, wherein
the manipulator assembly (300) including a plurality of electrically-operated actuation units (600) configured to actuate a plurality of control members associated with said medical device in response to a plurality of actuation control signals (210).

**Patentansprüche**

1. Vorrichtung zur Verwendung in einem Robotersteuerungssystem zum Manipulieren einer medizinischen Vorrichtung in Richtung eines Ziels, mit:

einer elektronischen Steuerungseinheit (ECU) (202), die mit einer Manipulatoranordnung (300) des Robotersteuerungssystems und mit einem Näherungssensor (236) gekoppelt ist;
einem computerlesbaren Speicher (206), der mit der ECU (202) gekoppelt ist; und
einer Steuerungslogik (228), die in dem Speicher (206) gespeichert ist, konfiguriert, um von der ECU (202) ausgeführt zu werden, wobei die Steuerungslogik (228) konfiguriert ist zum Erzeugen eines Betätigungssteuerungssignals (210) zum Steuern einer Betätigung einer Manipulatoranordnung (300) des Robotersteuerungssystems, um so die medizinische Vorrichtung in und bezüglich einer Mehrzahl von vordefinierten Näherungszonen zu navigieren, die jeweils Näherungskriterien aufweisen, die eine Spanne von Abständen von der Gewebegrenze repräsentieren,
wobei die Steuerungslogik (228) ferner konfiguriert ist zum Erzeugen des Betätigungssteuerungssignals (210) basierend auf einem Näherungssignal, das kennzeichnend ist für mindestens eine von einer Näherungsmetrik und einer Kontaktmetrik, bezogen auf einen Ort der medizinischen Vorrichtung relativ zu dem Gewebe (28) eines Patienten (30) und gemäß den vordefinierten Näherungszonen; **dadurch gekennzeichnet, dass**
die Steuerungslogik (226) ferner konfiguriert ist zum Erzeugen der Betätigungssteuerungssignale, um so die medizinische Vorrichtung mit reduzierter Ablenkung zu navigieren gemäß einem Ablenkungsschritt-Steuerungsparameter, der kennzeichnend ist für eine Reduzierung eines Ablenkungswinkels eines distalen Endes der medizinischen Vorrichtung, wenn das Näherungssignal die Kriterien einer Zone näher zu dem Gewebe erfüllt.

2. Vorrichtung nach Anspruch 1, bei der eine vorgeplante Bewegung der medizinischen Vorrichtung einen vorgeplanten Pfad und ein vorgeplantes Geschwindigkeitsprofil für die Vorrichtung während einer Bewegung entlang des Pfads aufweist, wobei die Steuerungslogik (228) konfiguriert ist zum Bewirken von Modifikationen der vorgeplanten Bewegung gemäß dem Näherungssignal und definierten Charakteristiken der Näherungszonen, wobei die Modifikationen aufweisen (i) eine reduzierte Geschwindigkeit relativ zu dem vorgeplanten Geschwindigkeitsprofil, (ii) ein Stoppen der Bewegung der Vorrichtung bevor der vorgeplante Pfad beendet ist, (iii) eine Bewegungsumkehr der Vorrichtung, oder (iv) eine Rückkehr der Vorrichtung zu einem vorherigen Ort innerhalb einer anderen Näherungszone.

3. Vorrichtung nach Anspruch 1 oder 2, ferner mit einer Benutzerschnittstellenlogik (226), die konfiguriert ist zum Anzeigen einer Ansicht einer Darstellung eines Bereichs des Körpers (30) und zum Empfangen einer Benutzereingabe bezüglich der Darstellung zur Spezifizierung von Geschwindigkeitsschwellenwerten, die den Näherungszonen zuzuordnen sind.

4. Vorrichtung nach einem der Ansprüche 1 oder 3, ferner mit einer Benutzerschnittstellenlogik (226), die konfiguriert ist zum Empfangen einer Benutzereingabe, um einen ersten Abstand bezüglich des Körpergewebes (28) zu spezifizieren zur Bestimmung einer ersten Grenze, wobei die Näherungszonen eine erste Näherungszone aufweisen, die zwischen der ersten Grenze und dem Körpergewebe (28) gebildet ist.

5. Vorrichtung nach Anspruch 4, bei der der erste Abstand eine Charakteristik ist, die zu der ersten Näherungszone gehört, wobei die Benutzerschnittstellenlogik (226) ferner konfiguriert ist zum Empfangen einer weiteren Benutzereingabe, um eine weitere Charakteristik zu spezifizieren, die zu der ersten Näherungszone gehört, umfassend einen Geschwindigkeitsschwellenwert oder eine Trajektorie, oder bei der die Benutzerschnittstellenlogik (226) ferner konfiguriert ist zum Empfangen einer weiteren Benutzereingabe, um einen zweiten Abstand bezüglich des Körpergewebes zu spezifizieren zur Bestimmung einer zweiten Grenze, wobei die Mehrzahl der Näherungszonen eine zweite Näherungszone aufweist, die zwischen der zweiten Grenze und der ersten Grenze gebildet ist.

6. Vorrichtung nach Anspruch 5, bei der die Mehrzahl der Näherungszonen eine dritte Näherungszone jenseits der ersten und zweiten Näherungszone aufweisen.

7. Vorrichtung nach Anspruch 5, bei der die Benutzerschnittstellenlogik (226) ferner konfiguriert ist zum Empfangen einer weiteren Benutzereingabe, um (i) einen Geschwindigkeitsschritt-Steuerungsparameter, der kennzeichnend ist für eine Reduzierung der Navigationsgeschwindigkeit der medizinischen Vorrichtung; (ii) den Ablenkungsschritt-Steuerungsparameter, der kennzeichnend ist für eine Reduzierung eines Ablenkungswinkels eines distalen Endes der medizinischen Vorrichtung; (iii) einen Translationsschritt-Steuerungsparameter, der kennzeichnend ist für eine Reduzierung einer Translationsgröße der medizinischen Vorrichtung, oder (iv) einen Entspannungsschritt, bei dem jegliche Spannungskraft oder Kompensationskraft, die auf die medizinische Vorrichtung wirkt, eine vollständige oder teilweise freigegebene Kraft ist, zu spezifizieren.

8. Vorrichtung nach Anspruch 1, bei der die Mehrzahl der Näherungszonen aufweist (i) eine erste Zone, die sich von einer Gewebegrenze mit einem ersten Abstand zu einer ersten Grenze erstreckt; (ii) eine zweite Zone, die sich von der ersten Grenze zu einer zweiten Grenze mit einem zweiten Abstand von der Gewebegrenze erstreckt; und (iii) eine dritte Zone, die sich jenseits der zweiten Grenze erstreckt;
wobei die Steuerungslogik konfiguriert ist zum Bestimmen, basierend auf dem Näherungssignal, in welcher von der ersten, zweiten und dritten Zone sich die medizinische Vorrichtung befindet, wobei die Steuerungslogik (226) ferner konfiguriert ist zum Erzeugen der Betätigungssteuerungssignale zum
Navigieren der medizinischen Vorrichtung normal gemäß einer vorgeplanten Bewegung mit einem Pfad und Geschwindigkeitskomponenten, wenn die medizinische Vorrichtung sich in der dritten Zone befindet;
Navigieren der medizinischen Vorrichtung mit einer reduzierten Geschwindigkeit relativ zu der vorgeplanten Geschwindigkeit, wenn sich die Vorrichtung in der zweiten Zone befindet; und
Unterbrechen der Navigation der medizinischen Vorrichtung vor Beendigung des vorgeplanten Pfads, wenn sich die medizinische Vorrichtung in der ersten Zone befindet.

9. Vorrichtung nach Anspruch 8, bei der das Robotersteuerungssystem eine Manipulatoranordnung aufweist, die Betätigungseinheiten hat, die konfiguriert sind zum Betätigen von einem oder von mehreren Steuerungselementen, die zu der medizinischen Vorrichtung gehören, in Antwort auf das Betätigungssteuerungssignal; und
die Steuerungslogik (226) konfiguriert ist zum Beenden einer Betriebsleistung für die Betätigungseinheiten, wenn sich die medizinische Vorrichtung in der ersten Zone befindet.

10. Vorrichtung nach Anspruch 9, bei der die Reduzierung der Geschwindigkeit bestimmt wird gemäß einem benutzerspezifizierten Geschwindigkeitsschritt-Steuerungsparameter.

11. Vorrichtung nach Anspruch 8, bei der die Steuerungslogik konfiguriert ist zum Erzeugen eines Alarms, wenn in einem benutzerspezifizierten Überwachungsmodus sich die medizinische Vorrichtung in der ersten oder der zweiten Zone befindet.

12. Vorrichtung nach Anspruch 10, bei der die Steuerungslogik (226) konfiguriert ist zum Verfolgen der Anzahl an Zeitpunkten, zu denen die medizinische Vorrichtung in die erste oder zweite Zone eintritt, wobei die Steuerungslogik (226) ferner konfiguriert ist zum Erhöhen eines Werts für den Geschwindigkeitsschritt-Steuerungsparameter, wenn die Anzahl an Zeitpunkten einen diagnostischen Schwellenwert überschreitet.

13. Vorrichtung nach Anspruch 1, bei der die medizinische Vorrichtung eine Elektrode (26) aufweist, und das Näherungssignal als Funktion einer komplexen Impedanz zwischen der Elektrode (26) und dem Gewebe (28) bestimmt wird.

14. Vorrichtung nach Anspruch 13, bei der das Näherungssignal einen elektrischen Kopplungsindex (ECI) aufweist und/oder eine Ableitung des ECI ist.

15. Robotersteuerungs- und Führungssystem zum Manipulieren einer medizinischen Vorrichtung in einem Körper (28) eines Patienten (30), das die Vorrichtung gemäß einem der Ansprüche 1 bis 14 aufweist, wobei die Manipulatoranordnung (300) eine Mehrzahl von elektrisch betreibbaren Betätigungseinheiten (600) aufweist, die konfiguriert sind zum Betätigen einer Mehrzahl von Steuerungselementen, die zu der medizinischen Vorrichtung gehören, in Antwort auf eine Mehrzahl von Betätigungssteuerungssignalen (210).

**Revendications**

1. Appareil pour utilisation dans un système de commande robotique pour manipuler un dispositif médical vers une cible, comprenant :

   une unité électronique de commande (ECU) (202) couplée à un ensemble manipulateur (300) du système de commande robotique et à un capteur de proximité (236) ;
   une mémoire lisible par ordinateur (206) couplée à ladite ECU (202) ; et
   une logique de commande (228) stockée dans ladite mémoire (206) configurée pour être exécutée par ladite ECU (202), ladite logique de commande (228) étant configurée pour produire un signal de commande d'actionnement (210) pour commander l'actionnement d'un ensemble manipulateur (300) du système de commande robotique afin de faire naviguer le dispositif médical dans et par rapport à une pluralité de zones de proximité prédéfinies ayant chacune un critère de proximité représentant une portée de distances à partir de la limite d'un tissu,

   ladite logique de commande (228) étant configurée en outre pour générer ledit signal de commande d'actionnement (210) sur la base d'un signal de proximité indiquant au moins l'une parmi une métrique de proximité et une métrique de contact en rapport avec un emplacement du dispositif médical par rapport à un tissu (28) d'un patient (30) et selon lesdites zones de proximité prédéfinies ;
   **caractérisé en ce que**
   ladite logique de commande (226) est configurée en outre pour générer lesdits signaux de commande d'actionnement afin de faire naviguer ledit dispositif médical avec une déviation réduite déterminée selon un paramètre de commande d'étape de déviation indiquant une diminution d'un angle de déviation d'une extrémité distale du dispositif médical quand le signal de proximité satisfait au critère d'une zone plus proche du tissu.

2. Appareil selon la revendication 1 dans lequel un déplacement préalablement planifié dudit dispositif médical inclut un trajet préalablement planifié et un profil de vitesse préalablement planifié pour le dispositif tandis qu'il se déplace le long dudit trajet, ladite logique de commande (228) étant configurée pour effectuer des modifications sur ledit déplacement préalablement planifié selon ledit signal de proximité et de caractéristiques définies desdites zones de proximité, dans lequel lesdites modifications incluent l'un parmi (i) une vitesse réduite par rapport audit profil de vitesse préalablement planifié, (ii) un arrêt du déplacement dudit dispositif avant la fin dudit trajet préalablement planifié, (iii) une inversion du déplacement dudit dispositif, et (iv) un retour dudit dispositif à un emplacement antérieur au sein d'une zone de proximité différente.

3. Appareil selon la revendication 1 ou 2 incluant en outre une logique d'interface utilisateur (226) configurée pour afficher une vue d'une représentation d'une partie du corps (30) et pour recevoir une entrée utilisateur par rapport à ladite représentation pour spécifier des seuils de vitesse à associer auxdites zones de proximité.

4. Appareil selon l'une quelconque des revendications 1 ou 3 incluant en outre une logique d'interface utilisateur (226) configurée pour recevoir une entrée utilisateur pour spécifier une première distance par rapport audit tissu corporel (28) pour déterminer une première limite, lesdites zones de proximité incluant une première zone de proximité qui est établie entre ladite première limite et ledit tissu corporel (28).

5. Appareil selon la revendication 4 dans lequel ladite première distance est une caractéristique associée à ladite première zone de proximité, ladite logique d'interface utilisateur (226) est configurée en outre pour recevoir une entrée utilisateur supplémentaire pour spécifier une caractéristique supplémentaire associée à ladite première zone de proximité, incluant l'un parmi un seuil de vitesse et une trajectoire, ou dans lequel ladite logique d'interface utilisateur (226) est configurée en outre pour recevoir une entrée utilisateur supplémentaire pour spécifier une seconde distance par rapport audit tissu corporel pour déterminer une seconde limite, ladite pluralité de zones de proximité incluant une deuxième zone de proximité établie entre ladite seconde limite et ladite première limite.

6. Appareil selon la revendication 5 dans lequel ladite pluralité de zones proximité inclut une troisième zone de proximité au-delà desdites première et deuxième zones de proximité.

7. Appareil selon la revendication 5 dans lequel ladite logique d'interface utilisateur (226) est configurée en outre pour recevoir une entrée utilisateur supplémentaire pour spécifier l'un parmi (i) un paramètre de commande d'étape de vitesse indiquant une diminution d'une vitesse de navigation du dispositif médical ; (ii) ledit paramètre de commande d'étape de déviation indiquant une diminution d'un angle de déviation d'une extrémité distale du dispositif médical ; (iii) un paramètre de commande d'étape de translation indiquant une diminution d'une quantité de translation du dispositif médical, et (iv) une étape de relaxation dans laquelle n'importe quelle force de tension ou force de compression conférée au dispositif médical est l'une parmi totalement libérée ou partiellement libérée.

8. Appareil selon la revendication 1 dans lequel ladite pluralité de zones de proximité inclut (i) une première zone se prolongeant à partir d'une limite de tissu sur une première distance vers une première limite ; (ii) une deuxième zone se prolongeant à partir de ladite première limite vers une seconde limite sur une seconde distance à partir de ladite limite de tissu ; et (iii) une troisième zone se prolongeant au-delà de ladite seconde limite ;
dans lequel ladite logique de commande est configurée pour déterminer, sur la base dudit signal de proximité, dans laquelle desdites première, deuxième et troisième zones ledit dispositif médical réside, ladite logique de commande (226) étant configurée en outre pour générer lesdits signaux de commande d'actionnement afin,
de faire naviguer ledit dispositif médical normalement selon un déplacement préalablement planifié ayant des composantes de trajet et de vitesse quand ledit dispositif médical est dans ladite troisième zone ;
de faire naviguer ledit dispositif médical avec une vitesse réduite par rapport à ladite vitesse préalablement planifiée quand ledit dispositif est dans ladite deuxième zone ; et
d'interrompre la navigation dudit dispositif médical avant la fin du trajet préalablement planifié quand le dispositif médical est dans la première zone.

9. Appareil selon la revendication 8 dans lequel ledit système de commande robotique inclut un ensemble manipulateur ayant des unités d'actionnement configurées pour actionner un ou plusieurs éléments de commande associés au dispositif médical en réponse audit signal de commande d'actionnement ; et dans lequel,
ladite logique de commande (226) est configurée pour amener une puissance de fonctionnement pour lesdites unités d'actionnement à être terminée quand ledit dispositif médical est dans ladite première zone.

10. Appareil selon la revendication 9 dans lequel ladite réduction de vitesse est déterminée selon un paramètre de commande d'étape de vitesse spécifié par un utilisateur.

11. Appareil selon la revendication 8 dans lequel ladite logique de commande est configurée pour générer une alerte quand dans un mode de surveillance spécifié par un utilisateur et que ledit dispositif médical est dans l'une parmi ladite première et ladite deuxième zone.

12. Appareil selon la revendication 10 dans lequel ladite logique de commande (226) est configurée pour suivre le nombre de fois que le dispositif médical entre dans l'une desdites première et deuxième zones, ladite logique de commande (226) étant configurée en outre pour augmenter une valeur pour ledit paramètre de commande d'étape de vitesse quand ledit nombre de fois dépasse un seuil de diagnostic.

13. Appareil selon la revendication 1 dans lequel ledit dispositif médical inclut une électrode (26), et dans lequel ledit signal de proximité est déterminé en fonction d'une impédance complexe entre ladite électrode (26) et ledit tissu (28).

14. Appareil selon la revendication 13 dans lequel ledit signal de proximité comprend un indice de couplage électrique (ECI) et/ou est un dérivé dudit ECI.

15. Système de commande et de guidage robotique pour manipuler un dispositif médical dans un corps (28) d'un patient (30), comprenant l'appareil selon l'une quelconque des revendications 1 à 14, dans lequel l'ensemble manipulateur (300) incluant une pluralité d'unités d'actionnement électriques (600) configurées pour actionner une pluralité d'éléments de commande associés audit dispositif médical en réponse à une pluralité de signaux de commande d'actionnement (210).

FIG.1

FIG.2

FIG.3a

FIG.3b

FIG.4a

FIG.4b

308,310

348

346

344

342

354

358

340

358

358

358

FIG.4c

FIG.5a

FIG.5b

EP 2 618 882 B1

FIG.6

29

FIG.7

EP 2 618 882 B1

FIG.8

FIG.9

FIG.10

FIG.11

EP 2 618 882 B1

FIG.12

FIG.13

OBTAIN PROXIMITY ZONE PARAMETERS — 1400

EVERY I/O CYCLE — 1402

OBTAIN UPDATED PROXIMITY (CONTACT) SENSOR DATA — 1404

MODE=OFF? — 1406 — YES

NO

PROXIMITY=GREEN ZONE? — 1408 — YES

NO

PROXIMITY=YELLOW ZONE? — 1410 — YES

NO

MODE=ACTIVE? — 1412 — NO

YES

CUT POWER — 1414

UPDATE DIAGNOSTICS AND AWAIT USER-INTERVENTION — 1416

MODE=ACTIVE? — 1418 — NO

YES

YELLOW ALERT — 1422

RED ALERT — 1424

1420

SCALE SPEED & DEFLECTION — 1426

FIG.14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 347811 A
- US 2009247993 A
- US 20100256558 A1
- US 20100069921 A1
- US 7263397 B
- US 6690963 B
- US 7386339 B
- US 7197354 B
- US 6233476 B
- US 7536218 B
- US 7848789 B
- US 75184310 A
- US 2009038597 W
- WO 2009120982 A
- US 933063 A
- US 347442 A
- WO 2009120948 A
- US 09568808 A
- US 20090163904 A
- US 46533709 A
- US 20090275827 A